# EUROPEAN PATENT SPECIFICATION

(11) **EP 3 863 622 B1**
(45) Date of publication and mention of the grant of the patent: **24.07.2024**
(21) Application number: 19791396.5
(22) Date of filing: 02.10.2019
(51) Int. Cl.: A61K 31/202, A61K 45/06, A61P 15/02, A61K 35/60

(54) **TREATMENT OF VULVOVAGINAL DISORDERS**
BEHANDLUNG VON VULVOVAGINALEN STÖRUNGEN
TRAITEMENT DE TROUBLES VULVOVAGINAUX

(30) Priority: 09.10.2018 US 201862743237 P; 22.10.2018 US 201862748875 P
(43) Date of publication of application: 18.08.2021
(73) Proprietor: University Of Rochester, Rochester, NY 14642 (US)
(72) Inventor: FOSTER, David, Rochester, NY 14642 (US); FALSETTA WOOD, Megan, L., Rochester, NY 14642 (US); PHIPPS, Richard, P., Rochester, NY 14642 (US)
(74) Representative: Brand Murray Fuller LLP
(86) International application number: PCT/US2019/054234
(87) International publication number: WO 2020/076578

(56) References cited:
- US-A- 5 912 006
- FALSETTA M L ET AL: "A much-needed model for the preclinical testing of new vulvodynia therapies", JOURNAL OF LOWER GENITAL TRACT DISEASE 20171001 LIPPINCOTT WILLIAMS AND WILKINS NLD, vol. 21, no. 4, Supplement 1, 1 October 2017 (2017-10-01), pages - S27 CONF, XP009517764, ISSN: 1526-0976
- DMITRIEVA NATALIA ET AL: "Resolvins RvD1 and 17(R)-RvD1 alleviate signs of inflammation in a rat model of endometriosis", FERTILITY AND STERILITY, ELSEVIER SCIENCE INC, NEW YORK, NY, USA, vol. 102, no. 4, 11 August 2014 (2014-08-11), pages 1191 - 1196, XP029049488, ISSN: 0015-0282, DOI: 10.1016/J.FERTNSTERT.2014.06.046
- MURINA FILIPPO ET AL: "Alpha Lipoic Acid Plus Omega-3 Fatty Acids for Vestibulodynia Associated With Painful Bladder Syndrome.", JOURNAL OF OBSTETRICS AND GYNAECOLOGY CANADA : JOGC = JOURNAL D'OBSTETRIQUE ET GYNECOLOGIE DU CANADA : JOGC 03 2017, vol. 39, no. 3, March 2017 (2017-03-01), pages 131 - 137, XP009517770, ISSN: 1701-2163
- CHARLES N. SERHAN ET AL: "The resolution code of acute inflammation: Novel pro-resolving lipid mediators in resolution", SEMINARS IN IMMUNOLOGY., vol. 27, no. 3, 1 May 2015 (2015-05-01), US, pages 200 - 215, XP055531711, ISSN: 1044-5323, DOI: 10.1016/j.smim.2015.03.004
- ML FALSETTA ET AL: "A review of the available clinical therapies for vulvodynia management and new data implicating proinflammatory mediators in pain elicitation", BJOG: AN INTERNATIONAL JOURNAL OF OBSTETRICS AND GYNAECOLOGY, vol. 124, no. 2, 17 June 2016 (2016-06-17), GB, pages 210 - 218, XP055653685, ISSN: 1470-0328, DOI: 10.1111/1471-0528.14157
- FALSETTA M L ET AL: "Specialized pro-resolving Mediators: A promising new therapeutic avenue for vulvodynia", JOURNAL OF LOWER GENITAL TRACT DISEASE 20191001 LIPPINCOTT WILLIAMS AND WILKINS NLD, vol. 23, no. 4, Supplement 1, 1 October 2019 (2019-10-01), XP009517765, ISSN: 1526-0976
- MYSRAYN Y. F. A. REIS ET AL: "Anti-Inflammatory Activity of Babassu Oil and Development of a Microemulsion System for Topical Delivery", EVIDENCE-BASED COMPLEMENTARY AND ALTERNATIVE MEDICINE, vol. 2017, 1 January 2017 (2017-01-01), pages 1 - 14, XP055653730, ISSN: 1741-427X, DOI: 10.1155/2017/3647801

## Description

### CROSS REFERENCE TO RELATED APPLICATION

This application claims priority to U.S. Provisional Application No. 62/743,237 filed on October 9, 2018 and to U.S. Provisional Application No. 62/748,875 filed on October 22, 2018.

### GOVERNMENT INTERESTS

This invention was made with government support under HD069313 awarded by National Institutes of Health. The government has certain rights in the invention.

### FIELD OF THE INVENTION

This invention relates to treating vulvovaginal disorders such as female reproductive tract irritation or/and inflammation as defined in the claims.

### BACKGROUND OF THE INVENTION

Vulvovaginal disorders refer to a range of diseases and conditions that affect the vulva and vagina. Some of the vulvovaginal disorders include irritation, such as pain and pruritus, from the female reproductive tract. Such pain or pruritus is a significant clinical problem for which there are few effective therapies. For example, localized provoked vulvodynia (LPV), which is characterized by acute and lasting pain in response to light touching of the vulvar vestibule (area immediately surrounding the vaginal opening), afflicts as many as 1 in 3 women within their lifetime and causes significant psychological distress and sexual dysfunction (Harlow, BL; Kunitz, CG; Nguyen, RH; Rydell, SA; Turner, RM; MacLehose, RF. Am J Obstet Gynecol 2014, vol. 210, pp. 40 e1-8). Therefore, LPV is a significant women's health issue. Vestibular pain is associated with a significant reduction in quality of life. Although women afflicted with LPV experience profound pain, they show no overt signs of disease such as lesions or infections (Sadownik, LA. Int J Womens Health 2014, vol. 6, pp. 437-49). The origins of disease are poorly understood, with few models available for the preclinical testing of potential new therapies (Falsetta, M.L., et al. Journal of Lower Genital Tract Disease 2017, vol. 21, no. 4, supplement 1). All currently available therapies only manage pain and psychological distress, but do not target the underlying biological causes of disease. There is a need for treatment of reproductive tract irritation, such pain and pruritus.

### SUMMARY OF INVENTION

The invention provides a pro-resolving mediator for use in a method of reducing or preventing lower genital tract irritation in a female subject, the method comprising administering an effective amount of the pro-resolving mediator topically to a treatment site of the subject's lower genital tract, wherein the irritation is associated with a genital tract inflammatory condition selected from the group consisting of localized provoked vulvodynia (LPV), lichen planus, lichen sclerosus, desquamative inflammatory vaginitis, atrophic vulvovaginitis associated with breast cancer, and chronic pruritus, and wherein the pro-resolving mediator is (1) selected from the group consisting of Resolvin D2, Resolvin D3, Resolvin D4, Resolvin D5, Resolvin E1, Maresin-1, epi-Maresin-1, Lipoxin A4, Protectin D1, Protectin DX, and DHA; or (2) a mixture comprising DHA, 14-HDHA, 17-HDHA, and 18-HEPE..

In particular, the pro-resolving mediator provides a means of reducing, preventing, or treating lower genital tract irritation (such as pain or pruritus) in a subject. An effective amount of the pro-resolving mediator is administered topically to a treatment site of the subject's lower genital tract.

In one embodiment, the genital tract inflammatory condition is LPV. The treatment site can comprise the vulvar vestibule, external vulva, vestibule, or vagina.

The pro-resolving mediator can be administered at 0.0001 mg/kg to 100 mg/kg to the treatment site. The pro-resolving mediator can be administered once a week, 2-3 times a week, once a day, twice a day, or three times a day. In certain examples, the subject may have a disease or condition (such as atrophic vulvovaginitis associated with breast cancer or others mentioned herein) already and the pro-resolving mediator may be administered before the subject is exposed to a secondary irritation-causing stimulation (e.g., intercourse and tampon usage).

The pro-resolving mediator can further comprise administering a second therapeutic agent (e.g., a second pro-resolving mediator, an anti-microbial agent or an antiviral agent) to the subject.

The details of one or more embodiments of the invention are set forth in the description below. Other features, objectives, and advantages of the invention will be apparent from the description and from the claims.

### BRIEF DESCRIPTION OF THE DRAWINGS

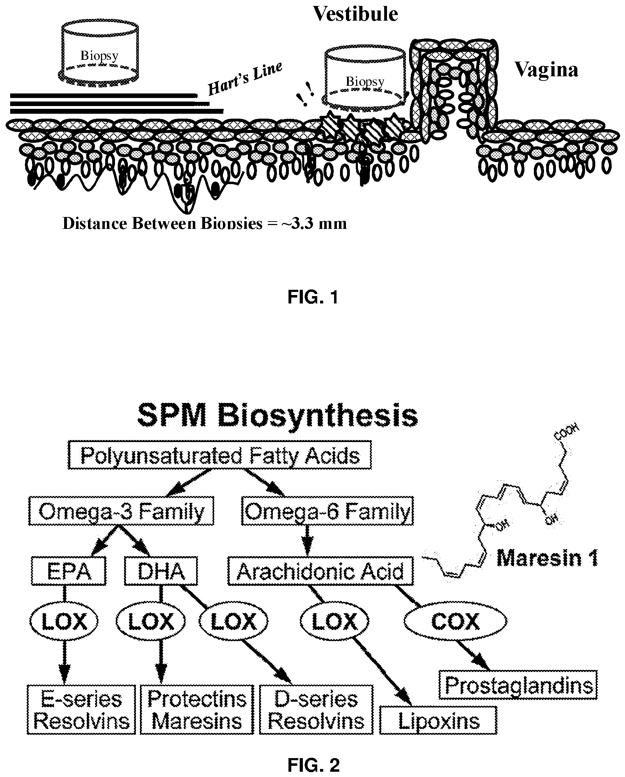

FIG. 1 is a diagram showing the sites of intense pain at the vestibule of LPV patients are in close proximity to non-painful sites of the external vulva and related biopsy sites.
FIG. 2 is a schematic of SPM biosynthesis. Specialized pro-resolving lipid mediators (SPM) are derived from polyunsaturated fatty acids (PUFAs) from the omega-3 and omega-6 families. PUFAs and other SPM precursors, such as, DHA and EPA (in the omega-3 family), go on to form SPMs, such as Maresins, Protectins, and Resolvins.

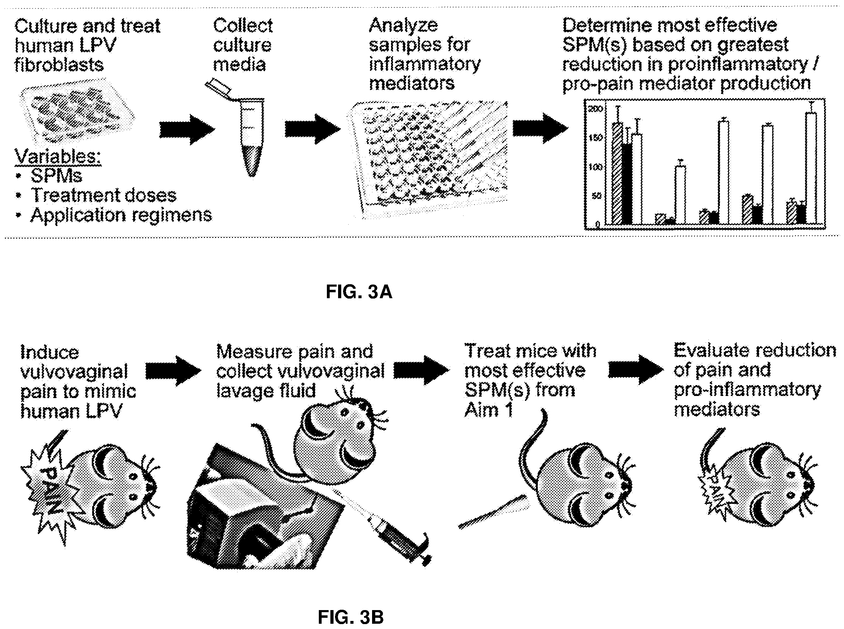

FIGs. 3A and 3B are a set of diagrams showing procedures for (A) investigating the ability to reduce proinflammatory and pro-pain mediator production form primary human cells in an *in vitro* LPV model and (B) evaluating the efficacy of SPMs in alleviating pain using a preclinical mouse model of LPV.

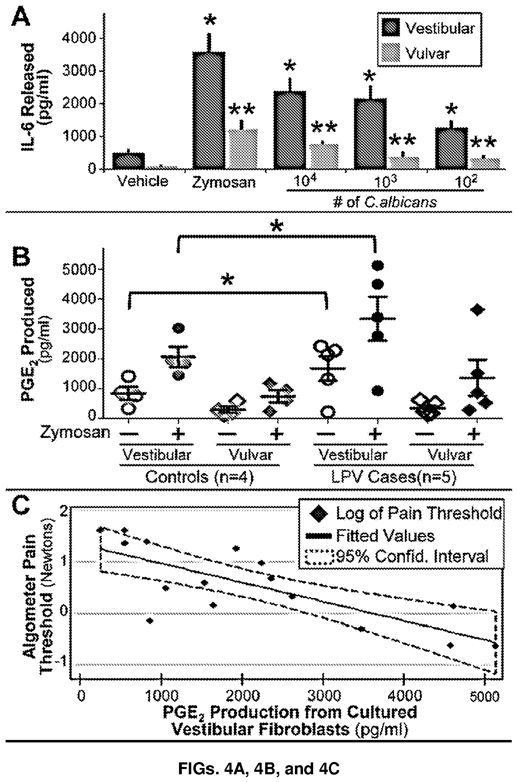

FIGs. 4A, 4B and 4C are a set of diagrams showing that inflammatory mediator production is elevated in vestibular cells from LPV patients compared to vulvar cells or control subjects. Panel A: IL-6 released in response to decreasing doses of live *C. albicans.* *p<0.05 vehicle vs. dose of *C. albicans* (for vestibular cells only), **p<0.05 vestibular vs. vulvar cells for a particular dose. ANOVA, n = 4. Vestibular cells show a strong response, while vulvar cells show no significant response to a dose up to 1000 times greater.³ Panel B: Cultured fibroblasts were stimulated with vehicle or zymosan (100 µg/ml) for 24 hours, then media were analyzed for prostaglandin E₂ (PGE₂). Zymosan induced a significant increase in PGE₂ over corresponding vehicle treatment. Vestibular fibroblasts from LPV case patients produced more PGE₂ compared to vestibular fibroblasts from control subjects. Mean +/- SEM. ANOVA *p < 0.05. Panel C: Scatter plot of fibroblast PGE₂ production plotted against log transformation of mucocutaneous pain threshold, performed before tissue sampling, from identical anatomical sites. Central line (solid red) represents fitted values of linear regression delimited by 95% confidence intervals (blue dotted line). t=2.58, p=0.04.

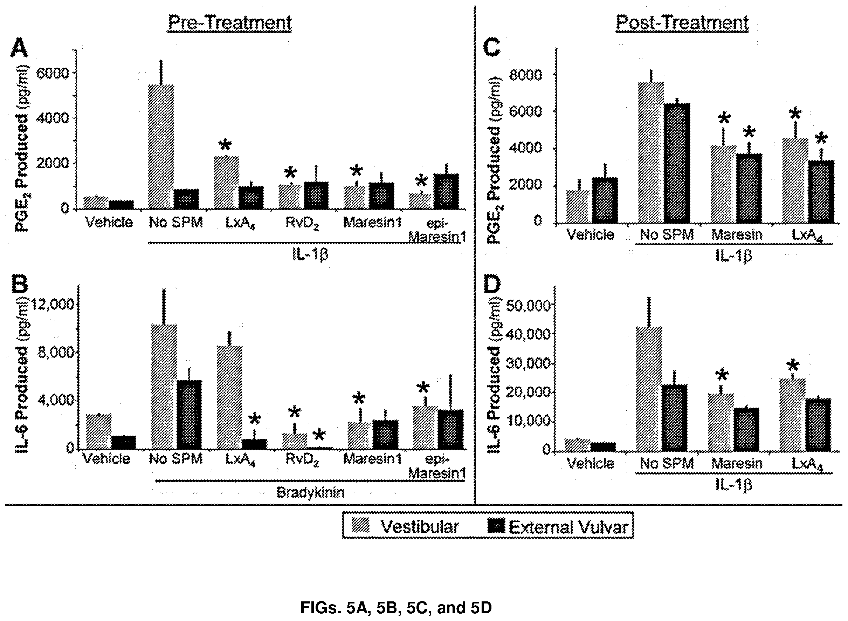

FIGs. 5A, 5B, 5C, and 5D are a set of diagrams showing that PGE₂ and IL-6 production is reduced by SPMs. Patient vestibular or vulvar fibroblasts were pre-treated for 10 hours with Lipoxin A₄ (LxA₄), Resolvin D₂ (RvD₂), (7R)-Maresin 1 or epi(7S)-Maresin 1 at a 5 nM concentration, then activated with IL-1β (Panel A; 10 pg/ml) or bradykinin (Panel B; 100 nM) for 48 hr. Culture media were collected and analyzed for PGE₂ (Panel A) or IL-6 (Panel B) content. Patient fibroblasts were also activated first with IL-1β for 30 min then treated with 5 nM (7R)-Maresin 1 or LXA₄ for 18 hours, followed by a booster dose for 6 hr. Culture media were collected and analyzed for PGE₂ (Panel C) or IL-6 (Panel D) content. Mean +/- SEM of n=3, ANOVA *p < 0.05 vs. activation only (no SPM). These results were consistent for several additional LPV patient strains.

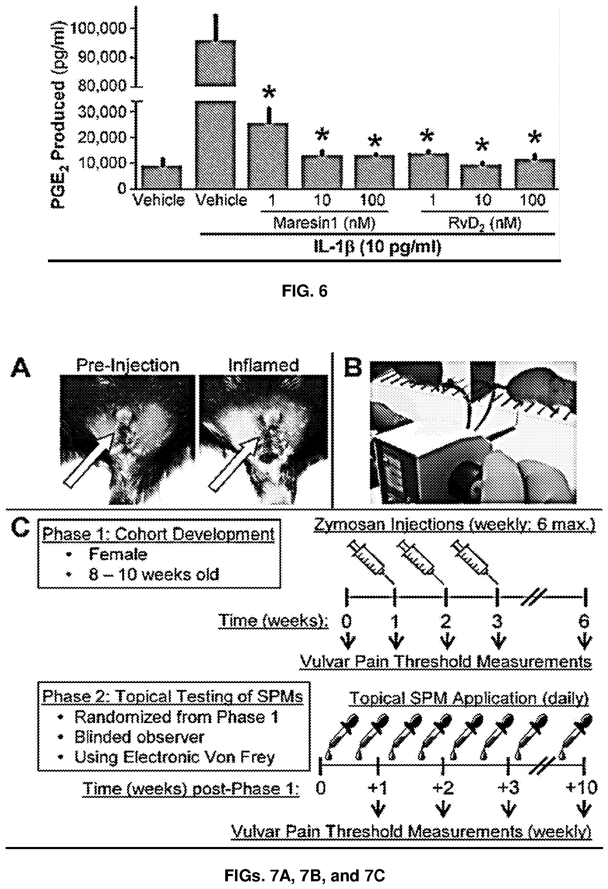

FIG. 6 is a diagram showing that SPMs inhibit PGE₂ production from mouse vulvar tissue. Mouse vulvar tissue (4 mm punch biopsies) was collected and bisected then pre-treated in culture medium with either Maresin 1 or RvD₂ at indicated concentrations for 18 hours, followed by an additional 18 hours stimulation with IL-1β (10 pg/ml). Culture medium was collected and analyzed for PGE₂ content. Mean +/- SEM; n = 3 replicate cultures. ANOVA * p < 0.05 vs. vehicle.
FIGs. 7A, 7B and 7C are a set of photographs and diagrams showing pain testing for *in vivo* mouse vulvodynia model. Panel A: After zymosan injection, inflammation and redness become apparent. Arrow indicates injection site. Panel B: Image showing how an electronic von Frey hair (Mousemet) is used to apply force to the mouse vulva. Panel C: Schematic of *in vivo* mouse model to establish then resolve vulvar allodynia.

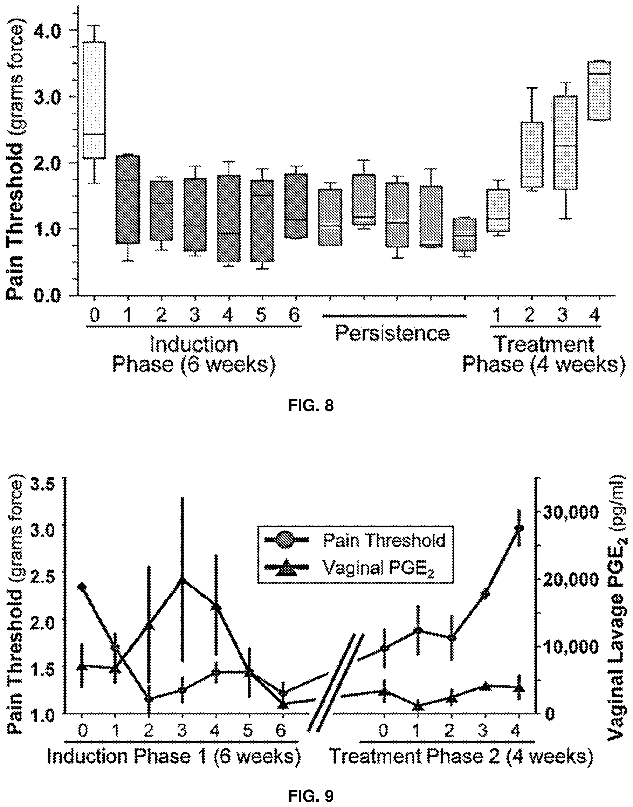

FIG. 8 is a diagram showing representative pain profile for a C57BL/6 mouse. This boxplot series shows median threshold values for a representative mouse over the induction, persistence, and treatment phases. Measures collected each week were tightly distributed, and thresholds were reduced >33% and maintained until treatment with Maresin 1, when values returned to baseline.
FIG. 9 is a diagram showing that PGE₂ in vaginal lavage is associated with pain threshold. After induction of allodynia for up to 6 weeks (indicated by reduced pain threshold, red line), Maresin 1 was applied to C57BL/6 mouse vulvas to reduce pain and increase pain threshold. PGE₂ content in vaginal lavage samples (blue line) rapidly increases during the induction phase, indicating pain-associated inflammation waning with treatment. SEM shown, n=8.

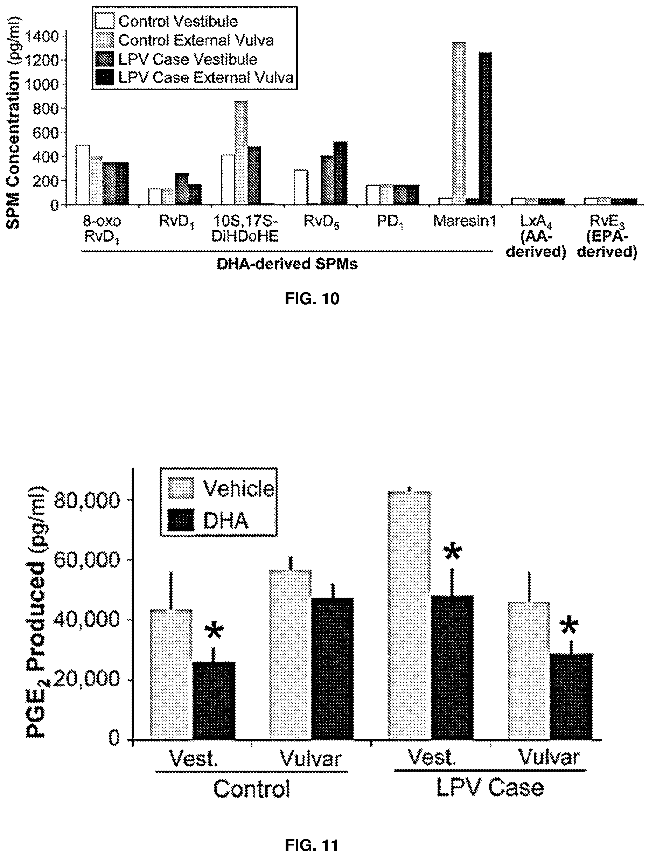

FIG. 10 is a diagram showing that SPMs are produced by vulvar fibroblasts. Fibroblasts were cultured for 48 h with IL-1β (10 pg/ml), then culture media were collected and frozen immediately on dry ice under argon gas for targeted lipidomic analysis. The predominating SPMs detected were derived from DHA.
FIG. 11 is a diagram showing that DHA supplementation also reduces proinflammatory mediator output, likely due to the production of SPMs. Vestibular or vulvar fibroblasts were pre-treated with DHA (200 nM) for 72 h, then activated with IL-1β (10 pg/ml) for 72 hr. Culture media were collected and analyzed for PGE₂ content. Mean +/-SEM of n=3 replicate treatment samples. ANOVA *p<0.05 vs. corresponding vehicle treatment.

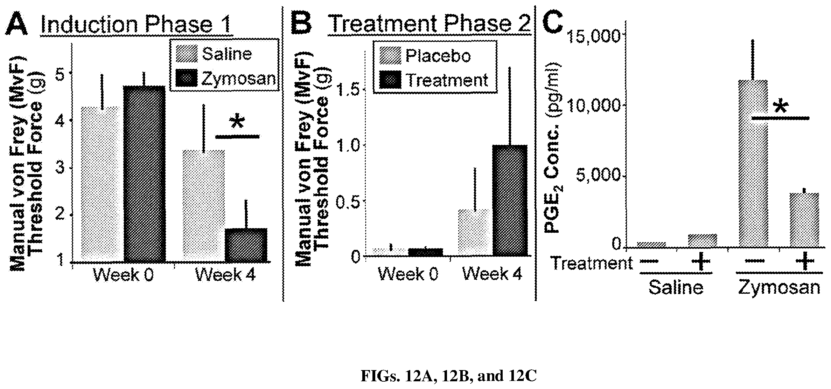

FIGs. 12A, 12B and 12C are a set of photographs showing manual von Frey assessment of pain threshold denotes improvement in threshold with treatment. Decreased thresholds in zymosan-treated mice after 4 weeks of injection reflects increased pain/sensitivity, Mean +/- SEM, n=8 saline, n=12 zymosan, ANOVA *p<0.05 (Panel A). Therapeutic treatment after the induction phase increased pain thresholds. Mean +/- SEM, n=7, p>0.05 (Panel B). Vulvovaginal lavage fluid was analyzed for PGE₂ content (Panel C). Mice receiving zymosan had elevated PGE₂ in their lavage fluid versus mice receiving saline injection. Furthermore, treated mice with allodynia had reduced PGE₂. Mean +/- SEM, n=7, ANOVA *p<0.05.

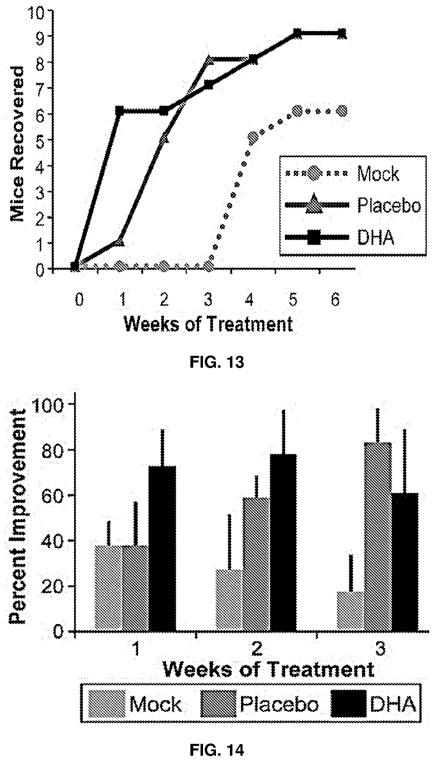

FIG. 13 is a diagram showing that mice recovering over time with treatment of DHA. Mice were considered to have recovered if they showed at least a 70% improvement in their pain threshold for two consecutive weeks. On the x-axis, week denotes the first week in a two-week period for which mice showed 70% improvement. Mice began to recover as early as week 1 in the DHA group. By week 3, most of the mice had recovered in the DHA and placebo groups, while no mice had recovered in the mock treated group.
FIG. 14 is a diagram showing percent improvement over first three weeks of treatment with DHA. The percent improvement in pain threshold (over lowest pre-treatment pain threshold) was greatest in the DHA group for the first two weeks. By week 3, percent improvement was similar between the DHA and placebo groups, but greater than the mock group. Mean +/- SEM.

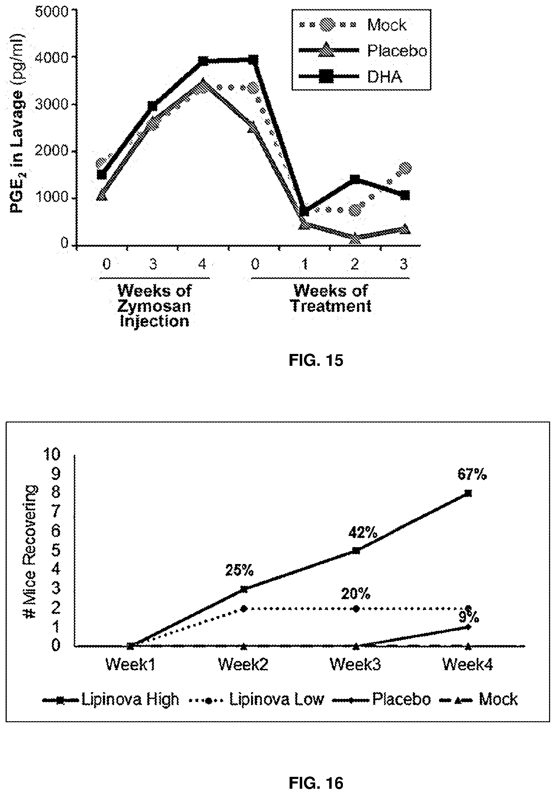

FIG. 15 is a diagram showing PGE₂ levels in vulvovaginal lavages. PGE₂ levels increased with zymosan injection and waned with treatment. During the first three weeks of treatment, levels dropped and remained low in the DHA and placebo groups.
FIG. 16 is a diagram showing mice recovering over time with treatment. Mice were considered to have recovered once they achieved thresholds that were 66% (+/- 0.5 g force) of their baseline threshold (prior to pain induction) for two consecutive weeks. On the x-axis, week denotes the treatment week; after week 2, mice were considered to have recovered if week 1 and 2 thresholds met the 66% criteria. The percentage of mice recovering is listed above the data points. Mice began to recover as early as week 2 in the LIPINOVA high and low group. By week 4, 8 (67%) mice in the LIPINOVA high group had recovered, while 2 had recovered in the low dose group. The only other mice recovering included 1 mouse in the placebo group. These data show that the LIPINOVA high dose is most effective in eliciting recovery.

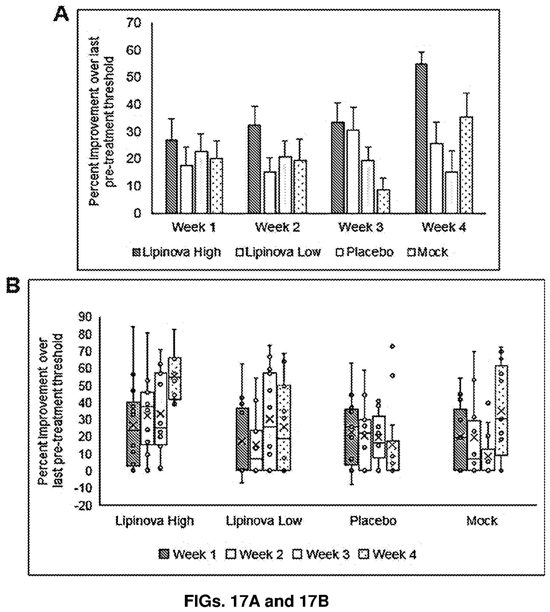

FIGs. 17A and 17B are diagrams showing percent improvement over four weeks of treatment. A) The data is graphed as the mean percent threshold for each treatment group +/-SEM. The percent improvement in pain threshold (over last pre-treatment pain threshold) was greatest in the LIPINOVA high group for all four weeks of treatment and gradually increased with time, showing >50% improvement by week 4. Although improvement was apparent in the controls and LIPINOVA low dose group, these values were lower than the LIPINOVA high group, and the differences between the LIPINOVA low dose and controls were marginal. B) The data is graphed as a box and whisker plot. Each dot represents the value for a single mouse, while the X represents the median. The box represents the upper and lower quartile, while the whiskers display the maximum and minimum values. This display shows that the medians are consistently higher for the LIPINOVA high group and at week 4 the median in the mock group is skewed by a few mice with particularly high threshold values that week.

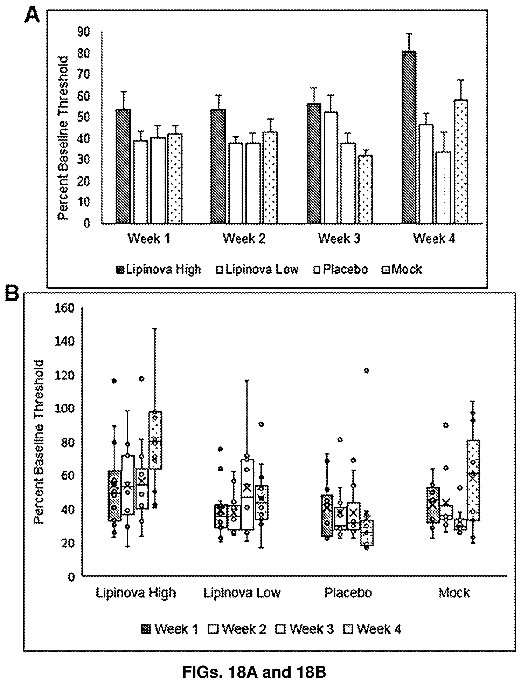

FIGs. 18A and 18B are diagrams showing percent baseline over four weeks of treatment. A) The data is graphed as the mean percent threshold for each treatment group +/-SEM. The percent pre-pain baseline scores were greatest in the LIPINOVA high group for all four weeks of treatment and reached the recovery value (for the entire group) by week 4. Although some recovery in baseline scores was apparent in the controls and LIPINOVA low dose group, these values were lower than the LIPINOVA high group, and the differences between the LIPINOVA low dose and controls were marginal. B) The data is graphed as a box and whisker plot. Each dot represents the value for a single mouse, while the X represents the median. The box represents the upper and lower quartile, while the whiskers display the maximum and minimum values. This display shows that the medians are consistently higher for the LIPINOVA high group and at week 4 the median in the mock group is skewed by a few mice with particularly high threshold values that week.

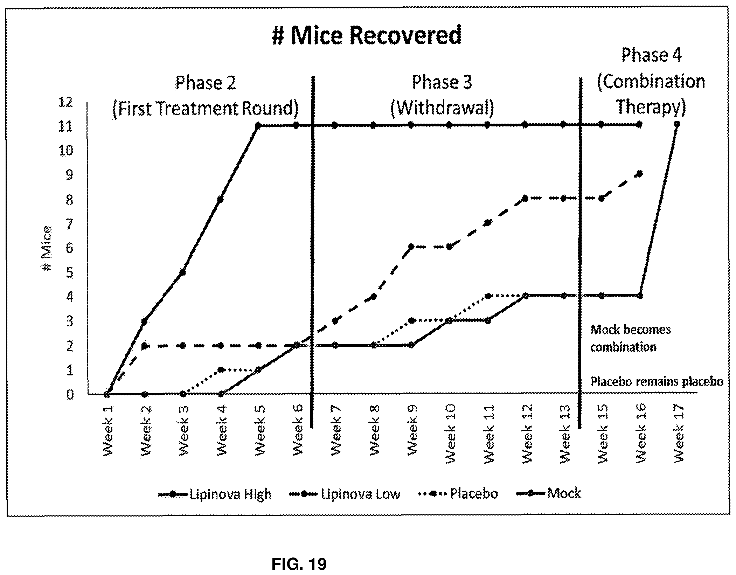

FIG. 19 is a diagram showing mice recovering over time with treatment. Mice were considered to have recovered if they returned to at least 70% of their baseline threshold for two consecutive weeks. On the x-axis, week denotes the study week; week 1 represents the first week of treatment. Mice began to recover as early as week 2 in the LIPINOVA groups. By week 5, most of the mice had recovered in the LIPINOVA high dose group, while only 2 recovered in the low dose group and 1 each in the mock and placebo groups. During phase 3 (withdrawal) a few more mice recovered in the low dose, placebo, and mock groups, and no mice showed a decrease in pain threshold over the withdrawal period. During withdrawal, greater numbers of mice recovered in the low dose versus placebo and mock groups. During phase 4 (combination therapy), all the mice recovered naturally, rendering us unable to assess the effects of treatment during this phase. n = 12 high and low dose group, n = 11 placebo and mock.

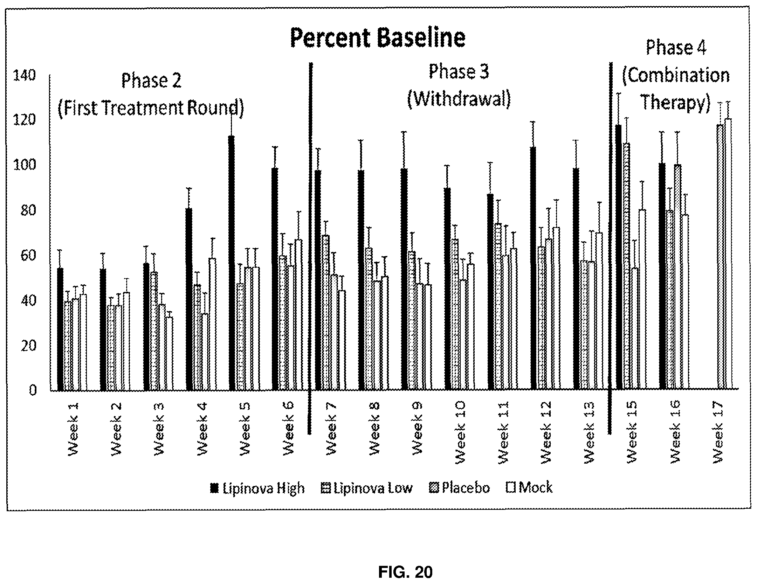

FIG. 20 is a diagram showing percent baseline threshold values. Values are presented at mean %baseline threshold value for all mice in the group +/- SEM. % baseline scores were consistently higher in the LIPINOVA high dose group. n = 12 high and low dose group, n = 11 placebo and mock group.

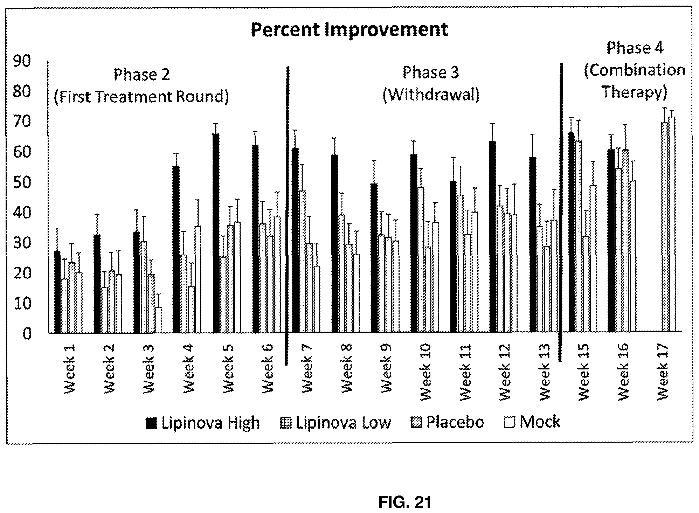

FIG. 21 is a diagram showing percent improvement over first three weeks of treatment. Values are presented as mean %improvement for all mice in the group +/- SEM. %improvement scores were consistently higher in the LIPINOVA high dose group. n = 12 high and low dose group, n = 11 placebo and mock group.

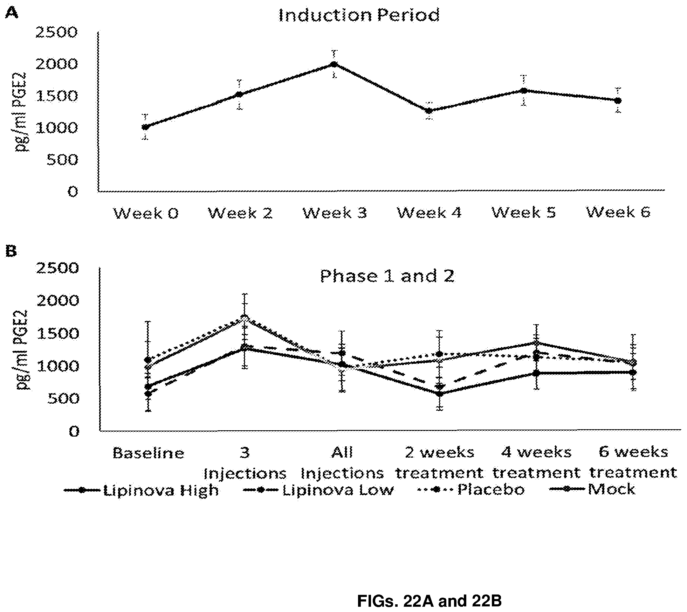

FIGs. 22A and 22B are diagrams showing PGE₂ levels throughout induction and treatment. Panel A depicts the average PGE₂ levels +/-SEM for all mice over the induction period. In panel A, where there are a greater number of data points to average (46 mice), the SEM is reduced compared panel B, where only the mice for each group are averaged (11-12 mice). Panel B depicts the PGE₂ values at baseline, at peak levels (3 weeks), after all injections were completed, and then at 2 week intervals during the treatment phase. The SEM values overlap and there is no statistically significant difference between groups, although the LIPINOVA high group appears to have lower levels of PGE₂ during treatment. n = 12 high and low dose group, n = 11 placebo and mock group.

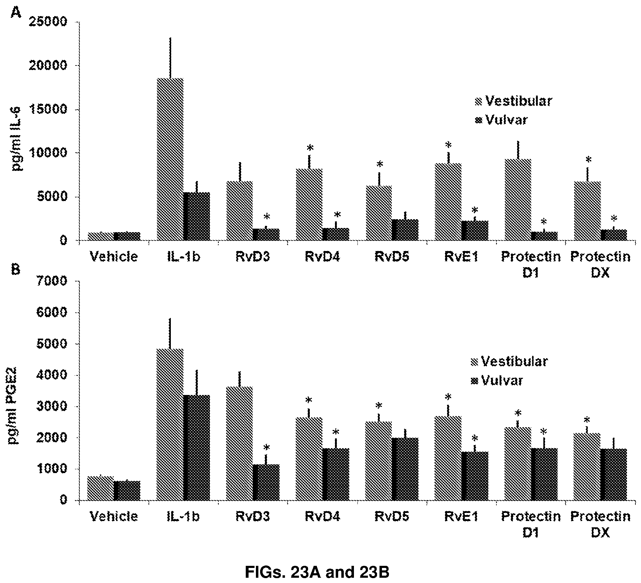

FIGs. 23A and 23B are diagrams showing six SPMs are effective in reducing IL-6 and PGE₂ production from fibroblasts when used as a pre-treatment and post-treatment. Patient vestibular or vulvar fibroblasts were pre-treated for 18 h with Resolvin D₃ (RvD₃), Resolvin D₄ (RvD₄), Resolvin D₅ (RvD₅), Resolvin E₁ (RvE₁), Protectin D1 or Protectin DX at a 5 nM concentration, pre-treated again for 30 min prior to activation, then activated with 10 pg/ml IL-1β for 48 h with a booster SPM dose at 24 h. Culture media were collected and analyzed for IL-6 (Panel A) and PGE₂ (Panel B). Mean +/- SEM of n=6 (two cases run in triplicate), ANOVA *p < 0.05 vs. activation only (IL-1b). All SPMs tested showed efficacy in reducing IL-6 and PGE₂ production when used a pre-treatment (before the onset of inflammation).

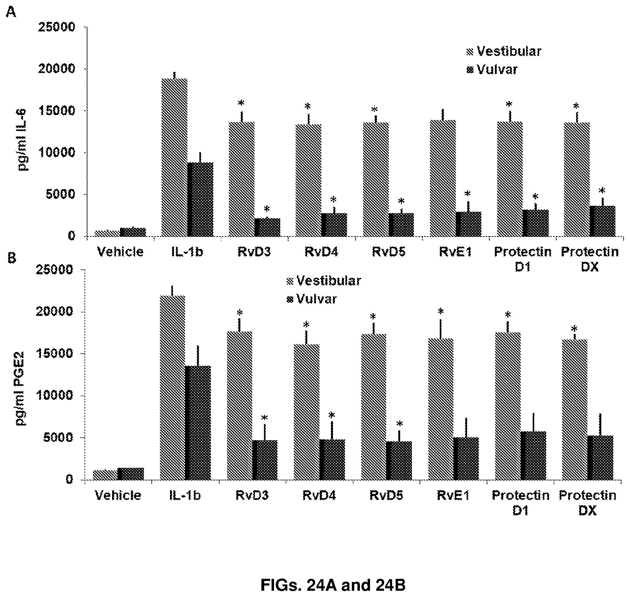

FIGs. 24A and 24B are diagrams showing that six SPMs are effective in reducing IL-6 and PGE₂ production from fibroblasts when used as a post-treatment. Patient vestibular or vulvar fibroblasts were activated first with IL-1β for 30 min then treated with RvD₃, RvD₄, RvD₅, RvEi, Protectin D1 or Protectin DX at a 5 nM concentration for 18 hours, followed by a booster SPM dose for 24 hr. Culture media were collected and analyzed for IL-6 (Panel A) and PGE₂ (Panel B) content. Mean +/- SEM of n=6 (two cases run in triplicate), ANOVA *p < 0.05 vs. activation only (IL-1b). All SPMs tested showed efficacy in reducing IL-6 and PGE₂ production, even after inflammation had been initiated, suggesting these SPMs would be effective in even in the presence of ongoing inflammation. The magnitude of reduction was not as great as pre-treatment, but equally significant as the reduction from pre-treatment regimens.

### DETAILED DESCRIPTION OF THE INVENTION

This invention is based, at least in part, on unexpected discoveries that fibroblasts isolated and cultured from sites of pain in LPV patients produce very high levels of pro-inflammatory and pro-pain mediators compared to "pain free" sites and that a class of molecules called pro-resolving mediators are effective against LPV.

LPV is the most common cause of longstanding dyspareunia (painful sexual intercourse) in premenopausal women, characterized by pain to light touch limited to the vulvar vestibule surrounding the vaginal opening. In women with LPV, chronic vestibular pain is crippling, impacting every aspect of life and exacerbating comorbidities, such as fibromyalgia and painful bladder. The devastating impact of LPV includes sexual dysfunction, infertility, depression, and even suicide. Yet, LPV etiology is unclear, and no effective medical therapy exists. This invention addresses this unmet need.

As disclosed herein, it was discovered that the vulvar vestibule expresses a unique inflammatory profile involving the elevated production of pro-pain and proinflammatory mediators, e.g., prostaglandin E₂ (PGE₂) and interleukin-6 (IL-6) by fibroblast strains isolated from the vestibule site (FIG. 1, "Vestibule"). Furthermore, elevated proinflammatory mediator release correlates with pain profiles in women. Therefore, effective therapeutics for LPV would ideally reduce proinflammatory signaling, while preserving the natural ability of these cells to respond to harmful proinflammatory stimuli. The investigation described herein has identified mechanisms by which hypersensitivity to certain inflammatory stimuli leads to heightened pain. Specifically, it was demonstrated that pain in LPV patients is directly correlated with the production of proinflammatory and pro-pain mediators from fibroblasts cultured from biopsies of painful sites (FIG. 1, "Vestibule").

As described herein, fibroblasts producing high levels of pro-pain and proinflammatory mediators can be isolated from patients at sites with intense, quantifiable pain. As they abundantly produce pro-pain mediators and maintain their relevant phenotypes in culture, the primary vestibular fibroblasts are valuable in modeling LPV and were used successfully here to identify new therapeutic agents that can be used to resolve atypical inflammatory mediator production in LPV patients that leads to regional pain. Therapeutic agents identified include pro-resolving mediators.

### Pro-resolving mediators

As used herein "pro-resolving mediator" refers to a compound or mixture as defined in the claims.

In aspects which are not encompassed by the wording of the claims, a "pro-resolving mediator" include a lipid-derived compound or substance that promotes the resolution of inflammation, *e.g.,* it can reduce one sign or symptom of inflammation in a cell or organism. In other aspects which are not encompassed by the wording of the claims, pro-resolving mediators include a class of lipids called "specialized pro-resolving mediators" (SPMs), their precursors, mixtures of different SPMs, mixtures of different SPM precursors, and mixtures of SPM and SPM precursor.

SPMs represent a class of pro-resolving, anti-pain and anti-inflammatory lipids naturally derived from omega-3 and omega-6 fatty acids (see, *e.g.,* FIG. 2) that help healing without compromising the body's ability to defend against inflammatory insults (e.g., infection or injury).⁵⁻⁷ SPMs are a genus with several families of potent endogenous bioactive products derived from precursors essential fatty acids EPA, DHA, arachidonic acid (ARA) and Docosapentaenoic acid (DPA) that are biosynthesized by positional and stereospecific incorporation of one, two or three molecules of molecular oxygen into a polyunsaturated fatty acid (PUFA) using EPA, DHA, ALA and DPA as substrates into a catalyzed reaction involving fatty acid lipoxygenases, cyclooxygenase type-2, when acetylated by aspirin, and several cytochrome P450 oxidases.

The term "specialized pro-resolving mediator" or SPM relates to a PUFA-derived enzymatically-oxygenated derivative that has potent anti-inflammatory and resolution-activating activity and that acts as endogenous regulator of the inflammatory response to bring an inflamed tissue back towards its non-inflamed and healthy state. SPMs act as endogenous receptor ligands or allosteric modulators to potently activate cellular responses that conceitedly activate anti-inflammatory actions and expedite, stimulate, and trigger resolution of inflammation. The term "SPM precursor" refers to an enzymatically oxygenated derivative of a PUFA that requires an additional enzymatic reaction to convert it to a SPM. A SPM precursor is a more proximate substrate for the endogenous formation of an SPM than the corresponding PUFA substrate itself.

The SPMs include several families of mediators, lipoxins, resolvins (*e.g.,* the E and D series), protectins and maresins. Examples of SPM include resolvin E1 (RvE1; 5S,12,18-trihydroxy-eicosa-6Z,8E,10E,14Z,16E-pentaenoic acid), 18S-resolvin E1 (18S-RvE1; 5S, 12R, 18S-trihydroxy-eicosa-6Z,8E, 10E, 14Z, 16E-pentaenoic acid), 20-hydroxy-RvE1 (5S,12R,18R,20-tetrahydroxy-eicosa-6Z,8E,10E,14Z,16E-pentaenoic acid), resolvin E2 (RvE2; 5S,18-dihydroxy-eicosa-6E,8Z,11Z,14Z,16E-pentaenoic acid), resolvin E3 (RvE3; 17,18R-dihydroxy-eicosa-5Z,8Z,11Z,13E,15E-pentaenoic acid), 18S-resolvin E3 (18S-RvE3; 17,18S-dihydroxy-eicosa-5Z,8Z,11Z,13E,15E-pentaenoic acid), 17,18-epoxy-eicosa-5Z,8Z,11Z,13E,15E-pentaenoic acid, lipoxin As (LXA₅; 5S,6R,15S-trihydroxy-eicosa-7E,9E,11Z,13E,17Z-pentaenoic acid), 15-epi-lipoxin A5 (LXA5; 5S,6R,15R-trihydroxy-eicosa-7E,9E,11Z,13E,17Z-pentaenoic acid), maresin 1 (MaRl; 7R,14S-docosa-4Z,8E,10E,12Z,16Z,19Z-hexaenoic acid), 7S-maresin 1 (7S-MaR1; 7S,14S-docosa-4Z,8E,10E,12Z,16Z,19Z-hexaenoic acid), 7S,14S-diHDHA (7S,14S-dihydroxy-docosa-4Z,8E,10Z,12E,16Z,19Z-hexaenoic acid), protectin D1 (PD1; 10R,17S-dihydroxy-docosa-4Z, 7Z, 11E, 13E, 15Z, 19Z-hexaenoic acid), 10S,17S-HDHA (10S,17S-dihydroxy-docosa-4Z,7Z,11E,13Z,15E,19Z-hexaenoic acid), 14S,21 S-diHDHA (14S,21 S-dihydroxy-docosa-4Z,7Z,10Z, 12E, 16Z, 19Z-hexaenoic acid), 14S,21R-diHDHA (14S,21R-dihydroxy-docosa-4Z,7Z,10Z,12E,16Z,19Z-hexaenoic acid), 14R,21 S-diHDHA (14R,21 S-dihydroxy-docosa-4Z,7Z,10Z,12E,16Z,19Z-hexaenoic acid), 14R,21 R-diHDHA (14R,21 R-dihydroxy-docosa-4Z,7Z,10Z,12E,16Z,19Z-hexaenoic acid), 13 S,14S-epoxy-DHA (13 S,14S-epoxy-docosa-4Z,7Z,9E,11E,16Z19Z-hexaenoic acid), 16,17S-diHDHA (16,17S-dihydroxy-docosa-4Z,7Z,10Z,12E,14E,19Z-hexaenoic acid), 16,17-epoxy-DHA (16,17-epoxy-docosa-4Z,7Z,10Z,12E,14E,19Z-hexaenoic acid), resolvin D1 (RvD1; 7S,8R,17S-trihydroxy-docosa-4Z,9E,11E,13Z,15E,19Z-hexaenoic acid), resolvin D2 (RvD2; 7S,16R,17S-trihydroxy-docosa-4Z,8E,10Z,12E,14E,19Z-hexaenoic acid), resolvin D3 (RvD3; 4S,11R,17S-trihydroxy-docosa-5Z,7E,9E, 13Z, 15E, 19Z-hexaenoic acid), resolvin D4 (RvD4; 4S,5,17S-trihydroxy-docosa-6E,8E,10Z,13Z,15E,19Z-hexaenoic acid), resolvin D5 (RvD5; 7S,17S-dihydroxy-docosa-5Z,8E,10Z,13Z,15E,19Z-hexaenoic acid), resolvin D6 (RvD6; 4S,17S-dihydroxy-docosa-5E,7Z,10Z,14Z,16E,19Z-hexaenoic acid), aspirin-triggered resolving D1 (AT-RvD1; 7S,8R,17R-trihydroxy-docosa-4Z,9E,11E,13Z,15E,19Z-hexaenoic acid), aspirin-triggered resolvin D2 (AT-RvD2; 7S,16R,17R-trihydroxy-docosa-4Z,8E,10Z,12E,14E,19Z-hexaenoic acid), aspirin-triggered resolvin D3 (AT-RvD3; 4S,11,17R-trihydroxy-docosa-5Z,7E,9E,13Z, 15E, 19Z-hexaenoic acid), aspirin-triggered resolvin D4 (AT-RvD4; 4S,5,17R-trihydroxy-docosa-6E,8E,10Z,13Z,15E,19Z-hexaenoic acid), aspirin-triggered resolvin D5 (AT-RvD5; 7S,17R-dihydroxy-docosa-5Z,8E,10Z,13Z,15E,19Z-hexaenoic acid), aspirin-triggered resolvin D6 (AT-RvD6; 4S,17R-dihydroxy-docosa-5E,7Z,10Z,14Z,16E,19Z-hexaenoic acid), 7S,17S-diHDPA n-3 (7S,17S-dihydroxy-docosa-8E,10Z,13Z,15Z,19Z-pentaenoic acid (ω-3)), lipoxin A₄ (LXA₄; 5S,6R,15S-trihydroxy-eicosa-7E,9E,11Z,13E-tetraenoic acid), 15-epi-lipoxin A₄ (15-epi-LXA₄; 5S,6R,15R-trihydroxy-eicosa-7E,9E, 11Z, 13E-tetraenoic acid), delta 12-prostaglandin J₂ (delta12-PGJ₂; 11-oxo-15S-hydroxy-prosta-SZ,9,12E-trienoic acid), 15-deoxy-delta12,14-prostaglandin J₂ (15-deoxy-delta12,14-PGJ₂; 11-oxo-prosta-5Z,9,12E,14E-tetraenoic acid), 11(12)-epoxy-eicosatetraenoic acid (11(12)-EpETE; 11(12)-epoxy-eicosa-5Z,8Z, 14Z, 17Z-tetraenoic acid), 17(18)-epoxy-eicosatetraenoic acid (17(18)-EpETE; 17(18-epoxy-eicosa-5Z,8Z,11Z,14Z-tetraenoic acid), 19(20)-epoxy-docosapentaenoic acid (19(20)-EpDPE; 19(20)-epoxy-docosa-4Z,7Z10Z, 13Z,16Z-pentaenoic acid), 10S,17S-HDPA n-6 (10S,17S-dihydroxy-docosa-4Z,7Z,11E,13Z,15E-pentaenoic acid), 7,17-HDPA n-6 (7,17-dihydroxy-docosa-4Z,8E,10Z,13Z,15E-pentaenoic acid), 7,14-HDPA n-6 (7,14-dihydroxy-docosa-4Z,8E,10Z,12Z,16Z-pentaenoic acid), 10S,17S-HDPA n-6 (10S,17S-dihydroxy-docosa-7Z,11E,13Z,15E,19Z-pentaenoic acid), and/or 7, 17-HDPA n-6 (7,17 dihydroxy-docosa-8E,10Z,13Z,15E,19Z-pentaenoic acid).

Examples of SPM precursors include 5S-HEPE (5S-hydroxy-eicosa-6E,8Z,11Z,14Z,17Z-pentaenoic acid); 11S-HEPE (11S-hydroxy-eicosa-5Z,8Z,12E,14Z,17Z-pentaenoic acid); 12S-HEPE (12S-hydroxy-eicosa-5Z,8Z,10E,14Z,17Z-pentaenoic acid); 12R-HEPE (12R-hydroxy-eicosa-5Z,8Z,10E,14Z,17Z-pentaenoic acid); 15S-HEPE (15S-hydroxy-eicosa-5Z,8Z,11Z,13E,17Z-pentaenoic acid); 4S-HDHA (4S-hydroxy-docosa-5E,7Z,10Z,13Z,16Z,19Z-hexaenoic acid); 7S-HDHA (7S-hydroxy-docosa-4Z,8E,10Z,13Z,16Z,19Z-hexaenoic acid); 10S-HDHA (10S-hydroxy-docosa-4Z,7Z,11E,13Z,16Z,19Z-hexaenoic acid); 11S-HDHA (11S-hydroxy-docosa-4Z, 7Z, 9E, 13 Z, 16Z, 19Z -hexaenoi c acid); 14S-HDHA (14S-hydroxy-docosa-4Z,7Z,10Z,12E,16Z,19Z-hexaenoic acid); 14R-HDHA (14R-hydroxy-docosa-4Z,7Z,10Z,12E,16Z,19Z-hexaenoic acid); 20S-HDHA (20S-hydroxy-docosa-4Z,7Z,10Z,13Z,16Z,19Z-hexaenoic acid); 17S-HDPAn-6 (17S-hydroxy-docosa-4Z,7Z,10Z,13Z,15E-pentaenoic acid); 14S-HDPAn-6 (14S-hydroxy-docosa-4Z,7Z,10Z,12E,16Z-pentaenoic acid); 10S-HDPAn-6 (10S-hydroxy-docosa-4Z,7Z,11E,13Z,16Z-pentaenoic acid); 17S-HDPAn-3 (17S-hydroxy-docosa-7Z,10Z,13Z,15E,19Z-pentaenoic acid); 14S-HDPAn-3 (17S-hydroxy-docosa-7Z,10Z,12E,16Z,19Z-pentaenoic acid); 10S-HDPAn-6 (10S-hydroxy-docosa-7Z,11E,13Z,16Z,19Z-pentaenoic acid); 15S-HETE (15S-hydroxy-eicosa-5Z,8Z,11Z,13E-tetraenoic acid); and/or 15R-HETE (15R-hydroxy-eicosa-5Z,8Z,11Z,13E-tetraenoic acid).

According to the invention, the pro-resolving mediator may be (1) selected from the group consisting of Resolvin D2, Resolvin D3, Resolvin D4, Resolvin D5, Resolvin E1, Maresin-1, epi-Maresin-1, Lipoxin A4, Protectin D1, Protectin DX, and DHA; or (2) a mixture comprising DHA, 14-HDHA, 17-HDHA, and 18-HEPE,

Other examples, present for illustrative purposes only, of pro-resolving mediator include, but are not limited to a Resolvin; Resolvin D₁; Resolvin D₆; aspirin-triggered Resolvin; aspirin-triggered Resolvin D₁; Protectin D1; 17-HDHA; 14-HDHA; 13-HDHA; 7-HDHA; 4-HDHA; a Lipoxin; a Lipoxin analog; Lipoxin A₄; a Lipoxin A₄ analog; Lipoxin B₄; 5,15-dihydroxyeicosatetraenoic acid; Thromboxane B₂; 15-hydroxyeicosatetraenoic acid (HETE); 12-HETE; 11-HETE; 5-HETE; Resolvin Es (*e.g.,* Resolvin E1, 18S-Resolvin E1, 20-hydroxy-Resolvin E1, Resolvin E2, 18S-Resolvin E3,); Lipoxin A₅; Lipoxin B₅; 5,15-dihydroxyeicosapentaenoic acid; 18-hydroxyeciosapentaenoic acid (HEPE); 15-HEPE; 12-HEPE; 5-HEPE; Annexin A1; a multi-region agent having a lipoxin region (see U.S. Pat. No. 5,441,951); aspirin-triggered lipid mediators, aspirin-triggered (ω-3) lipid mediators; 16-dimethyl-LXA₄; 15-epi-LXA₄; benzo-LXA₄ analogs; 9 (o-[9,12]-benzo-15-epi-LXA(4) methyl ester; and analogs or mimetics thereof. Such mediators and methods of producing them are described in, for example, U.S. Pat. Nos. 7,615,576; 5,441,951; 6,887,901; 7,737,178; 7,595,341; 7,378,444; 7,585,856; 6,703,423; 7,700,650; 7,812,054; 7,132,451; U.S. Patent Publications 2010/0105772; 2010/0105773; 2009/0156673; 2006/0293288; 2003/0166716, 2008/0312323, 20140079631, 20150126602, 20180200375, and 2018/0256597; and Serhan et al. FASEB Journal 2012 26.

Certain SPMs and SPM precursors are found in oils derived from natural sources such as fish, crustaceae (krill), algae (long chain ω-3 PUFA-producing algae), mollusks, and from other organisms containing long chain ω-3 PUFA. These oils and their fractions containing or enriched with one or more such SPMs or SPM precursors as defined in the claims can also be used in this invention. SPMs and SPM precursors may derive from any of the following omega-3 PUFA or omega-6 PUFA: Hexadecatrienoic acid (HTA), a-Linolenic acid (ALA), Stearidonic acid (SDA), Nonadecatetraenoic acid, Eicosatrienoic acid, Eicosatetraenoic acid, Eicosapentaenoic acid (EPA), Heneicosapentaenoic acid, Docosapentaenoic acid (DPA), Docosahexaenoic acid (DHA), Tetracosapentaenoic acid, and Tetracosahexaenoic acid.. These fatty acids may give rise to SPM precursors and SPMs through enzymatic oxygenation.

In aspects not encompassed by the wording of the claims, pro-resolving mediators also include, in addition to the SPMs and SPM precursors listed above, other mono-, di-, and tri-hydroxylated and epoxygenated derivatives of the above mentioned polyunsaturated fatty acids, which possess anti-inflammatory and proresolving activities. These derivatives can be found to be present and enriched in oils obtained from organism which contain long chain ω-3 PUFA including fish, crustaceae, algae, mollusks, and marine organisms, plants, microbial organisms, as well as transgenic organisms endowed with the enzymatic capacity to form long chain ω -3 PUFA. Likewise, additional precursors of known SPMs and novel SPMs may be identified and enriched in such oils. In addition, the SPMs and SPM precursors may be present as esters and amides. The esters can be natural esters such as triglycerides, diglycerides, monoglycerides, and phospholipids, as well as esters prepared during the industrial processes commonly employed in the fish oil industry permitting the concentration of EPA and DHA from crude and refined fish oils, in particular the form of ethyl esters.

Any SPM, SPM precursor, or mixtures of SPMs and SPM precursors that are found in oils obtained from long chain ω-3 PUFA-containing organisms can be enriched or concentrated employing extraction and separation methods known in the art. Examples include distillation technologies, and chromatographic fractionation and separation technologies.

Pro-resolving mediators of this invention such as SPMs resolve inflammation and pain without impairing normal host defense. The resolution of inflammation and pain, once thought to be a passive process during which proinflammatory signaling tapers off, is now known to be an active process mediated by SPMs. SPMs actively reduce proinflammatory signaling, promote bacterial clearance, reduce pain, and accelerate wound healing. SPMs can be naturally produced by the human body, have virtually no toxicity, and several are in clinical trial for other afflictions. SPMs are not traditional anti-inflammatory agents and are not immunosuppressive; they do not affect the body's ability to sense and respond to infection or injury.

As disclosed herein, pro-resolving mediators can be ideal therapeutic agents for LPV, as they foster wound healing, promote bacterial clearance, and reduce pain and proinflammatory signaling. Although SPMs have not been clinically tested as an LPV therapy, evidence presented here supports that pro-resolving mediators are efficacious in reducing pain-provoking proinflammatory mediator production and in turn, reduce LPV-associated pain *in vivo.*

Pro-resolving mediators, including molecules such as SPMs naturally produced by the human body promote bacterial clearance, reduce pain, and accelerate wound healing. Using an *in vitro* model described herein, inventors identified a number of SPMs (such as Resolvin D₂, Maresin-1, epi-Maresin-1, and Lipoxin A₄) highly effective in reducing IL-6 and PGE₂ production in cells when administered prior to inflammatory stimulation. Furthermore, at least two of these SPMs (Maresin-1 and Lipoxin A4) are highly effective in reducing IL-6 and PGE₂ in already activated cells, suggesting they are effective throughout the entire disease process. In addition, it was found that supplementing fibroblasts with SPM precursor, docosahexaenoic acid, also reduced proinflammatory signaling and supported the production of SPMs, detected by targeted lipidomic analysis. The results described herein suggest that SPMs or their precursors are effective LPV therapies.

As also disclosed herein, a robust and reproducible mouse model of LPV was developed to assess therapeutic intervention against vulvar pain (the first of its kind). The model couples real-time proinflammatory mediator quantification with mechanical pain testing via an electronic von Frey to monitor pain and inflammation over time. Inventors were able to establish stable allodynia in mice, lasting more than several months. During allodynia induction, it was found that pain thresholds decreased, while proinflammatory mediator levels (*e.g.,* PGE₂) increased within collected vulvovaginal fluids, consistent our *in vitro* findings. Inventors then treated mice daily with topical Maresin-1 or DHA, which increased pain thresholds, while suppressing PGE₂ levels. The *in vitro* and *in vivo* findings disclosed herein suggest that topical application of SPMs can reduce vulvar pain and inflammation and would represent an ideal therapy for LPV.

### Formulations and Uses

The pro-resolving mediator of the invention are for use in a method of reducing or preventing lower genital tract irritation in a female subject, the method comprising administering an effective amount of the pro-resolving mediator topically to a treatment site of the subject's lower genital tract, wherein the irritation is associated with a genital tract inflammatory condition selected from the group consisting of localized provoked vulvodynia (LPV), lichen planus, lichen sclerosus, desquamative inflammatory vaginitis, atrophic vulvovaginitis associated with breast cancer, and chronic pruritus.

In aspects not encompassed by the wording to the claims, the pro-resolving mediator compounds described above and related compositions are useful in methods of treating various inflammatory disorders or conditions, such as an irritation associated with inflammation. To that end, the earliest indicator found for increased vulvodynia risk of development is a woman's recognition of prolonged pain/irritation following intercourse (Reed BD, et al., Journal of Women's Health 2012;21:1139-43). Therefore, the pro-resolving mediator compounds described above and related compositions can be used as a primary prevention modality of vulvodynia development if it is applied by women when they recognize this as a problem.

The compounds or compositions of the invention are administered topically. In aspects not encompassed by the wording of the claims, the compounds or compositions can be administered in a therapeutically effective amount by any of the accepted modes of administration. Suitable dosage ranges depend upon numerous factors such as the severity of the disease to be treated, the age and relative health of the subject, the potency of the compound used, the route and form of administration, the indication towards which the administration is directed, and the preferences and experience of the medical practitioner involved. One of ordinary skill in the art of treating such diseases will be able, without undue experimentation and in reliance upon personal knowledge and the disclosure of this application, to ascertain a therapeutically effective amount of the pro-resolving mediator compounds of the present disclosure for a given disease. Thus, the compounds or compositions of the present disclosure can be administered as pharmaceutical formulations including those suitable for topical, vaginal, oral (including buccal and sub-lingual), rectal, nasal, pulmonary, or parenteral (including intramuscular, intraarterial, intrathecal, subcutaneous and intravenous) administration or in a form suitable for administration by inhalation or insufflation. In certain aspects, the manner of administration is topical, vaginal, or transdermal using a convenient daily dosage regimen which can be adjusted according to the degree of affliction.

In particular, topical and/or transdermal treatment using the compounds or compositions is preferred for local control of disease states and inflammatory cascade states for reducing or preventing lower genital tract pain in a subject, such as LPV, while insuring that any unwanted side effects are minimized and curtailed.

To that end, the pharmaceutical compositions of the present disclosure can be suitable for topical administration. In that case, the pharmaceutical compositions comprise one or more pro-resolving mediators, a pharmaceutically acceptable topical carrier, and optionally a permeation enhancer. In some aspects, the permeation enhancer can comprise a base. The base can be present at a concentration sufficient to provide a formulation pH in the range of approximately 7.5 to 13.0. The pharmaceutical composition can be aqueous. The aqueous pharmaceutical composition can be a cream, gel, lotion, paste, or solution.

Various skin-permeation enhancing agents are known in the art and can be used in this invention. Examples of suitable enhancers include, but are not limited to, ethers such as diethylene glycol monoethyl ether (available commercially as TRANSCUTOL) and diethylene glycol monomethyl ether; surfactants such as sodium laurate, sodium lauryl sulfate, cetyltrimethylammonium bromide, benzalkonium chloride, Poloxamer (231, 182, 184), Tween (20, 40, 60, 80), and lecithin (U.S. Pat. No. 4,783,450); alcohols such as ethanol, propanol, octanol, benzyl alcohol, and the like; polyethylene glycol and esters thereof such as polyethylene glycol monolaurate (PEGMI,; see, *e.g.,* U.S. Pat. No. 4,568,343); amides and other nitrogenous compounds such as urea, dimethylacetamide (DMA), dimethylformamide (DMF), 2-pyrrolidone, 1-methyl-2-pyrrolidone, ethanolamine, diethanolamine and triethanolamine; terpenes; alkanones; and organic acids, particularly citric acid and succinic acid. AZONE^{®} and sulfoxides such as DMSO and C₁₀ MSO may also be used.

Other suitable enhancers include those lipophilic co-enhancers typically referred to as "plasticizing" enhancers, *i.e.,* enhancers that have a molecular weight in the range of about 150 to 1000, an aqueous solubility of less than about 1 wt. %, preferably less than about 0.5 wt. %, and most preferably less than about 0.2 wt. %. The Hildebrand solubility parameter of plasticizing enhancers is in the range of about 2.5 to about 10, preferably in the range of about 5 to about 10. Such enhancers are described in, *e.g.,* U.S. Pat. No. 6,586,000, and WO 01/43775. Preferred lipophilic enhancers are fatty esters, fatty alcohols, and fatty ethers. Examples of specific and most preferred fatty acid esters include methyl laurate, ethyl oleate, propylene glycol monolaurate, propylene glycerol dilaurate, glycerol monolaurate, glycerol monooleate, isopropyl n-decanoate, and octyldodecyl myristate. Fatty alcohols include, for example, stearyl alcohol and oleyl alcohol, while fatty ethers include compounds wherein a diol or triol, preferably a C₂-C₄ alkane diol or triol, are substituted with one or two fatty ether substituents. Additional permeation enhancers are known in the art of topical drug delivery. See, *e.g.,* Percutaneous Penetration Enhancers, Smith et al., editors (CRC Press, 1995).

A formulation described herein may be in any form suitable for topical application, for example to the skin (*e.g.,* the external vulva, vestibule, or vagina) and surrounding tissues. It may comprise, for example, a cream, lotion, solution, gel, ointment, paste, plaster, paint, bioadhesive, or the like, and/or may be prepared to contain liposomes, micelles, and/or microspheres. Such a formulation may be aqueous, *i.e.,* contain water, or may be nonaqueous and optionally used in combination with an occlusive overlayer so that moisture evaporating from the body surface is maintained within the formulation upon application to the body surface and thereafter.

Formulations of the invention may optionally contain a pharmaceutically acceptable viscosity enhancer and/or film former. A viscosity enhancer increases the viscosity of the formulation to inhibit its spread beyond the site of application. Balsam Fir (Oregon) is an example of a pharmaceutically acceptable viscosity enhancer. A film former, when it dries, forms a protective film over the site of application. The film inhibits removal of the active ingredient and keeps it in contact with the site being treated. An example of a film former that is suitable for use in this invention is Flexible Collodion, USP. As described in Remington, The Science and Practice of Pharmacy, 19th Ed. (Easton, Pa.: Mack Publishing Co., 1995), at page 1530, collodions are ethyl ether/ethanol solutions containing pyroxylin (a nitrocellulose) that evaporate to leave a film of pyroxylin. A film former may act additionally as a carrier. Solutions that dry to form a film are sometimes referred to as paints.

Ointments, as is well known in the art of pharmaceutical formulation, are semisolid preparations that are typically based on petrolatum or other petroleum derivatives. The specific ointment base to be used, as will be appreciated by those skilled in the art, is one that will provide for optimum drug delivery, and, preferably, will provide for other desired characteristics as well, *e.g.,* emolliency or the like. As with other carriers or vehicles, an ointment base should be inert, stable, nonirritating and nonsensitizing. As explained in Remington: The Science and Practice of Pharmacy, 19th Ed. (Easton, Pa.: Mack Publishing Co., 1995), at pages 1399-1404, ointment bases may be grouped in four classes: oleaginous bases; emulsifiable bases; emulsion bases; and water-soluble bases. Oleaginous ointment bases include, for example, vegetable oils, fats obtained from animals, and semisolid hydrocarbons obtained from petroleum. Emulsifiable ointment bases, also known as absorbent ointment bases, contain little or no water and include, for example, hydroxystearin sulfate, anhydrous lanolin and hydrophilic petrolatum. Emulsion ointment bases are either water-in-oil (W/O) emulsions or oil-in-water (O/W) emulsions, and include, for example, cetyl alcohol, glyceryl monostearate, lanolin, and stearic acid. Preferred water-soluble ointment bases are prepared from polyethylene glycols of varying molecular weight; again, see Remington: The Science and Practice of Pharmacy for further information.

Creams, as also well known in the art, are viscous liquids or semisolid emulsions, either oil-in-water or water-in-oil. Cream bases are water-washable, and contain an oil phase, an emulsifier, and an aqueous phase. The oil phase, also called the "internal" phase, is generally comprised of petrolatum and a fatty alcohol such as cetyl or stearyl alcohol. The aqueous phase usually, although not necessarily, exceeds the oil phase in volume, and generally contains a humectant. The emulsifier in a cream formulation is generally a nonionic, anionic, cationic, or amphoteric surfactant.

As will be appreciated by those working in the field of pharmaceutical formulation, gels are semisolid, suspension-type systems. Single-phase gels contain organic macromolecules distributed substantially uniformly throughout the carrier liquid, which is typically aqueous, but also, preferably, contain an alcohol and, optionally, an oil. Preferred "organic macromolecules," *i.e.,* gelling agents, are crosslinked acrylic acid polymers such as the "carbomer" family of polymers, *e.g.,* carboxypolyalkylenes that may be obtained commercially under the CARBOPOL. Also preferred are hydrophilic polymers such as polyethylene oxides, polyoxyethylene-polyoxypropylene copolymers, and polyvinylalcohol; cellulosic polymers such as hydroxypropyl cellulose, hydroxyethyl cellulose, hydroxypropyl methylcellulose, hydroxypropyl methylcellulose phthalate, and methyl cellulose; gums such as tragacanth and xanthan gum; sodium alginate; and gelatin. In order to prepare a uniform gel, dispersing agents such as alcohol or glycerin can be added, or the gelling agent can be dispersed by trituration, mechanical mixing or stirring, or combinations thereof.

Lotions are preparations to be applied to the skin surface without friction, and are typically liquid or semiliquid preparations in which particles, including the active agent, are present in a water or alcohol base. Lotions are usually suspensions of solids, and preferably, for the present purpose, comprise a liquid oily emulsion of the oil-in-water type. Lotions are preferred formulations for treating large body areas, because of the ease of applying a more fluid composition. It is generally necessary that the insoluble matter in a lotion be finely divided. Lotions will typically contain suspending agents to produce better dispersions as well as compounds useful for localizing and holding the active agent in contact with the skin, e.g., methylcellulose, sodium carboxymethyl-cellulose, or the like.

Pastes are semisolid dosage forms in which the active agent is suspended in a suitable base. Depending on the nature of the base, pastes are divided between fatty pastes or those made from a single-phase aqueous gels. The base in a fatty paste is generally petrolatum or hydrophilic petrolatum or the like. The pastes made from single-phase aqueous gels generally incorporate carboxymethylcellulose or the like as a base.

Plasters are comprised of a pasty mixture that is spread on the body, either directly or after being saturated into a base material such as cloth. Medications, including the bases of the invention, may be dissolved or dispersed within the plaster to make a medicated plaster.

Bioadhesives are preparations that adhere to surfaces of body tissues. Polymeric bioadhesive formulations are well known in the art; see, for example, Heller et al., "Biodegradable polymers as drug delivery systems," in Chasin, M. and Langer, R., eds.: Dekker, New York, pp. 121-161 (1990); and U.S. Pat. No. 6,201,065. Suitable non-polymeric bioadhesives are also known in the art, including certain fatty acid esters (U.S. Pat. No. 6,228,383).

Formulations described in this invention may also be prepared with liposomes, micelles, and microspheres. Liposomes are microscopic vesicles having a lipid wall comprising a lipid bilayer, and can be used as drug delivery systems herein as well. Generally, liposome formulations are preferred for poorly soluble or insoluble pharmaceutical agents. Liposomal preparations for use in the instant invention include cationic (positively charged), anionic (negatively charged) and neutral preparations. Cationic liposomes are readily available. For example, N[1-2,3-dioleyloxy)propyl]-N,N,N-triethylammonium (DOTMA) liposomes are available under the tradename LIPOFECTIN^{®}. (GIBCO BRL, Grand Island, N.Y.). Similarly, anionic and neutral liposomes are readily available as well, e.g., from Avanti Polar Lipids (Birmingham, Ala.), or can be easily prepared using readily available materials. Such materials include phosphatidyl choline, cholesterol, phosphatidyl ethanolamine, dioleoylphosphatidyl choline (DOPC), dioleoylphosphatidyl glycerol (DOPG), dioleoylphoshatidyl ethanolamine (DOPE), among others. These materials can also be mixed with DOTMA in appropriate ratios. Methods for making liposomes using these materials are well known in the art.

Micelles are known in the art to be comprised of surfactant molecules arranged so that their polar head groups form an outer spherical shell, while the hydrophobic, hydrocarbon chains are oriented towards the center of the sphere, forming a core. Micelles form in an aqueous solution containing surfactant at a high enough concentration so that micelles naturally result. Surfactants useful for forming micelles include, but are not limited to, potassium laurate, sodium octane sulfonate, sodium decane sulfonate, sodium dodecane sulfonate, sodium lauryl sulfate, docusate sodium, decyltrimethylammonium bromide, dodecyltrimethylammonium bromide, tetradecyltrimethylammonium bromide, tetradecyltrimethylammonium chloride, dodecylammonium chloride, polyoxyl 8 dodecyl ether, polyoxyl 12 dodecyl ether, nonoxynol 10 and nonoxynol 30. Micelle formulations can be used in conjunction with the present invention either by incorporation into a topical or transdermal delivery system, or into a formulation to be applied to a target site (e.g., vestibule) and surrounding tissues.

Microspheres, similarly, may be incorporated into the present formulations and drug delivery systems. Like liposomes and micelles, microspheres essentially encapsulate a drug or drug-containing formulation. Microspheres are generally, although not necessarily, formed from synthetic or naturally occurring biocompatible polymers, but may also be comprised of charged lipids such as phospholipids. Preparation of microspheres is well known in the art and described in the pertinent texts and literature.

Various additives known in the art may be included in the topical formulations. For example, solvents, including relatively small amounts of alcohol, may be used to solubilize certain formulation components. The present formulations may also include conventional additives such as opacifiers, antioxidants, fragrance, colorants, gelling agents, thickening agents, stabilizers, surfactants, and the like. Other agents may also be added, such as antimicrobial agents, to inhibit growth of microbes such as bacteria, yeasts, and molds. Exemplary antimicrobial agents are typically selected from the group consisting of the methyl and propyl esters of p-hydroxybenzoic acid (*i.e.,* methyl and propyl paraben), sodium benzoate, sorbic acid, imidurea, and combinations thereof.

In one aspect not encompassed by the wording of the claims, the pro-resolving mediator compounds described above and related compositions are useful in methods of treating various inflammatory disorders or conditions. Varieties or combinations of this therapy include, though are not limited to the following exemplary applications: a topical/transdermal spray using a radiating pump dispenser; a topical/transdermal salve/balm rubbed into the treated area; a topical/transdermal wound cleansing rinse; a topical/transdermal roll-on for pain relief; an impregnated mini-sponge individually hermetically sealed with said composition that can be reconstituted with water; a wound powder composed of micronized, freeze dried material, and a time-released epidermal/topical patch for staged and sequential delivery of said composition for site-specific application.

The therapeutic composition may preferably be administered as needed. For example, for severe conditions, about 1-4 times per day on a daily basis can be used. In addition, the therapeutic composition may alternatively be administered on a weekly, bi-weekly, triweekly, weekly or monthly basis until the condition is treated or remediated as desired. Furthermore, the administration may initially begin on a daily basis and then, in response to clinical improvement, transition to a weekly, monthly, etc. administration. Rather than being used solely as a treatment aid, the composition of the present invention may also be used to maintain a user in pain free condition.

In certain embodiments, the effective dose of a composition comprising one or more pro-resolving mediators as described herein can be administered to a patient once. In certain embodiments, the effective dose of a composition comprising a pro-resolving mediator can be administered to a patient repeatedly. Patients can be administered a therapeutic amount of a composition comprising a pro-resolving mediator at 0.0001 mg/kg to 100 mg/kg, such as 0.5 mg/kg, 1.0 mg/kg, 2.0 mg/kg, 2.5 mg/kg, 5 mg/kg, 10 mg/kg, 15 mg/kg, 20 mg/kg, 25 mg/kg, 30 mg/kg, 40 mg/kg or 50 mg/kg. A composition comprising a pro-resolving mediator can be administered over a period of time, such as over a 5-minute, 10-minute, 15-minute, 20-minute, or 25-minute period. The administration is repeated, for example, on a regular basis, such as hourly for 3 hours, 6 hours, 12 hours or longer or such as biweekly (i.e., every two weeks) for one month, two months, three months, four months or longer. After an initial treatment regimen, the treatments can be administered on a less frequent basis. For example, after administration biweekly for three months, administration can be repeated once per month, for six months or a year or longer. Administration of a composition comprising a pro-resolving mediator can reduce levels of a marker or symptom of, for example, inflammation by at least 10%, at least 15%, at least 20%, at least 25%, at least 30%, at least 40%, at least 50%, at least 60%, at least 70%, at least 80% or at least 90% or more.

Treating LPV and other female genital tract conditions entails vaginal or perivaginal administration. To that end, vaginal or perivaginal dosage forms may include vaginal suppositories, creams, ointments, liquid formulations, pessaries, tampons, gels, pastes, foams or sprays. The suppository, cream, ointment, liquid formulation, pessary, tampon, gel, paste, foam or spray for vaginal or perivaginal delivery comprises a therapeutically effective amount of the selected active agent and one or more conventional nontoxic carriers suitable for vaginal or perivaginal drug administration. The vaginal or perivaginal forms of the present invention may be manufactured using conventional processes as disclosed in Remington: The Science and Practice of Pharmacy, supra (see also drug formulations as adapted in U.S. Pat. Nos. 6,515,198; 6,500,822; 6,417,186; 6,416,779; 6,376,500; 6,355,641; 6,258,819; 6,172,062; and 6,086,909). The vaginal or perivaginal dosage unit may be fabricated to disintegrate rapidly or over a period of several hours. The time period for complete disintegration may be in the range of from about 10 minutes to about 6 hours, e.g., less than about 3 hours.

The pro-resolving mediator described above can be included in other suitable compositions or kits. Examples of such compositions and kits include a birth control device or agent, a feminine sanitary product such as a douche, sanitary pad or, preferably a tampon, a vaginal or an anal suppository, or an enema, all of which may provide with one or more other therapeutic agents (*e.g.,* an antimicrobial agent, anti-viral agent, and anti-STD agent), and all of which may be provided as sustained release compositions (*e.g.,* in a sustained release device).

In aspects not encompassed by the wording of the claims, the pro-resolving mediators disclosed herein are for use in a method of treating various vulvovaginal disorders, including pain, pruritus, and other female genital tract conditions.

The pain (vulvovaginal pain) can be caused by a specific disorder: an infectious disorder (such as recurrent candidiasis and herpes); an inflammatory disorder (such as lichen sclerosus, lichen planus, anorectal Crohn's, and desquamative inflammatory vaginitis); a neoplastic disorder, (such as Paget disease, squamous cell carcinoma, and atrophic vulvovaginitis associated to breast cancer); a neurologic disorder (such as postherpetic neuralgia, nerve compression or injury, and neuroma); trauma (such as female genital cutting and obstetric); a latrogenic disorder (such as postoperative, chemotherapy, vulvovaginal Graft Vs. Host, and radiation); and hormonal deficiencies (such as genitourinary syndrome of menopause or vulvovaginal atrophy, and lactational amenorrhea).

Graft-versus-host (GvH) disease is a sequela of hematopoietic stem cell transplantation (HSCT) in up to 80% of transplant recipients and over that past 20 years HSCT procedures have increased 3-fold (Gratwahl et al., Blood 100 (2002), pp. 2374-2386). GvH develops when the graft's immunocompetent T cells recognize alloantigens displayed by the host's antigen-presenting cells and react against the immunocompromised host. Genital GVHD has been estimated to occur in up to 48% of women after HSCT (Zantomio et al., Bone Marrow Transplant. 2006 Oct;38(8):567-72), and has been considered a common sign of chronic GVHD by oncologists. Vaginal symptoms, include dryness, itching, burning, soreness, introital pain as well as stenosis and fibrosis are described. Physical findings range from mild cases of erythema plus leukorrhea to more severe findings of strictures, fibrosis and in some, complete obliteration of the vaginal and vulvar anatomy. To date, there has been no specific effective therapy for this condition.

In addition, the pain can be caused by vulvodynia, which is a vulvar pain of at least three-month duration, without clear identifiable cause that may have potential associated factors. Vulvodynia is a chronic discomfort or pain, consisting of burning, stinging, irritation, and rawness on the vulva. This pain can be: generalized (diffuse vulvar burning or irritation); localized (pain at a specific area, such as the vestibule, and clitoris, vestibulodynia or clitorodynia, respectively); mixed (localized and generalized); provoked (e.g., insertional, contact); spontaneous; mixed (provoked and spontaneous); localized provoked vulvodynia (LPV); onset (primary or secondary); and show a temporal pattern (intermittent, persistent, constant, immediate, delayed).

The pro-resolving mediator of the invention are for use in a method of reducing or preventing lower genital tract irritation in a female subject, the method comprising administering an effective amount of the pro-resolving mediator topically to a treatment site of the subject's lower genital tract, wherein the irritation is associated with a genital tract inflammatory condition selected from the group consisting of localized provoked vulvodynia (LPV), lichen planus, lichen sclerosus, desquamative inflammatory vaginitis, atrophic vulvovaginitis associated with breast cancer, and chronic pruritus.

Lichen planus may present as one of two types: (1) "classic", consisting of sharply demarcated, flat-topped plaques on oral and genital membranes and (2) "erosive", consisting of an erosive, erythematous lesion originating in the vestibule and variably extending up the vaginal canal. Erosive lichen Planus is commonly characterized, symptomatically, by chronic spontaneous burning pain.

Lichen sclerosus is visually characterized by depigmentation, a loss of mucocutaneous markings, and submucosal hemorrhage. Reduced elasticity of the skin surface may result in fissuring at the perineal body. Lichen sclerosus may involve the labia minora, clitoris, interlabial sulcus, and inner portion of labia majora and perianal areas as well. Circumferential depigmentation of the vaginal introitus and the adjacent perianal region with lichen sclerosus has been characterized by the descriptive term "keyhole distribution".

Desquamative inflammatory vaginitis is characterized by burning pain, visible inflammation and increased vaginal discharge on clinical exam, and evidence of parabasal cells, microscopically. A key diagnostic hallmark is the finding of parabasal cells with inflammation in the presence of adequate estrogenization, and absence of infectious etiology on microscopic study, or other laboratory method.

Atrophic vulvovaginitis associated with breast cancer is characterized by burning pain and painful intercourse. On clinical exam, there is loss of vaginal rugal architecture, dryness, and visible pallor to the mucosa. The use of topical estrogen for treatment has been controversial in this group of cancer survivors.

Other conditions that can be treated include chronic pruritus. The problem of pruritus can be as debilitating as pain in many with lichen sclerosus and lichen planus. It is mediated by a similar neural fiber (c fiber) as allodynia although by microneurography pruritus is mediated through a distinct neural subset. The mediators (inflammosomes) of pruritus appear to be similar to the pain mediators

As used herein, the terms "treat," "treatment," "treating," or "amelioration" when used in reference to a disease, disorder or medical condition, refer to therapeutic treatments for a condition, wherein the object is to reverse, alleviate, ameliorate, inhibit, slow down or stop the progression or severity of a symptom or condition (such as pain). The term "treating" includes reducing or alleviating at least one adverse effect or symptom of a condition. Treatment is generally "effective" if one or more symptoms or clinical markers are reduced. Alternatively, treatment is "effective" if the progression of a condition is reduced or halted. That is, "treatment" includes not just the improvement of symptoms (such as pain) or markers (such as cytokines), but also a cessation or at least slowing of progress or worsening of symptoms that would be expected in the absence of treatment. Beneficial or desired clinical results include, but are not limited to, alleviation of one or more symptom(s), diminishment of extent of the deficit, stabilized (*i.e.,* not worsening) state of inflammation, delay or slowing of inflammation, and amelioration or palliation of inflammation.

The term "topical," as used herein, refers to the administration of the compositions of the invention to the skin and underlying tissues, as well as to administration to the mucosa and underlying tissues.

The terms "decrease," "reduce," "reduced", "reduction", "decrease," and "inhibit" are all used herein generally to mean a decrease by a statistically significant amount relative to a reference. However, for avoidance of doubt, "reduce," "reduction" or "decrease" or "inhibit" typically means a decrease by at least 10% as compared to a reference level and can include, for example, a decrease by at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 98%, at least about 99%, up to and including, for example, the complete absence of the given entity or parameter as compared to a reference level, or any decrease between 10-99% as compared to the absence of a given treatment.

When the terms "prevent", "preventing", and "prevention" are used herein in connection with a given treatment for a given condition, they mean that the treated patient either does not develop a clinically observable level of the condition at all, or develops it more slowly and/or to a lesser degree than he/she would have absent the treatment. These terms are not limited solely to a situation in which the patient experiences no aspect of the condition whatsoever. For example, a treatment will be said to have "prevented" the condition if it is given during exposure of a patient to a stimulus that would have been expected to produce a given manifestation (such as pain) of the condition, and results in the patient's experiencing fewer and/or milder symptoms of the condition than otherwise expected. For example, a treatment can "prevent" inflammation by resulting the patient's displaying only mild overt symptoms of the inflammation; it does not imply that there must have been no inflammation or no production of pro-inflammatory cytokines, inflammation mediators and/or the related downstream cellular events.

As used herein, the term "enriched" refers to a composition (*e.g.,* an oil) containing SPMs and/or SPM precursors when it contains a higher level of SPMs and/or SPM precursors than the source from which it was made.

As used herein, the phrase "therapeutically effective amount", "effective amount" or "effective dose" refers to an amount that provides a therapeutic or aesthetic benefit in the treatment, prevention, or management of, for example, pain, inflammation or wound healing, *e.g.* an amount that provides a statistically significant decrease in at least one symptom, sign, or marker of pain, inflammation, and/or wound healing. Determination of a therapeutically effective amount is well within the capability of those skilled in the art. Generally, a therapeutically effective amount can vary with the subject's history, age, condition, sex, as well as the severity and type of the medical condition in the subject, and administration of other pharmaceutically active agents.

The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio. As used herein, the term "pharmaceutical composition" refers to the active agent in combination with a pharmaceutically acceptable carrier commonly used in the pharmaceutical industry.

As used herein, a "subject" means a human or an animal. Preferably, the subject is a mammal. The mammal can be a human, non-human primate, mouse, rat, dog, cat, horse, or cow, but are not limited to these examples. Mammals other than humans can be advantageously used, for example, as subjects that represent animal models of, for example, inflammation. In addition, the methods described herein can be used to treat domesticated animals and/or pets.

A subject can be one who has been previously diagnosed with or identified as suffering from or having a condition in need of treatment (*e.g.,* inflammation or other pain-causing conditions) or one or more complications related to such a condition, and optionally, but need not have already undergone treatment for a condition or the one or more complications related to the condition. Alternatively, a subject can also be one who has not been previously diagnosed as having a condition in need of treatment or one or more complications related to such a condition. For example, a subject can be one who exhibits one or more risk factors for a condition or one or more complications related to a condition or a subject who does not exhibit risk factors. For example, a female subject may be treated as described herein to remediate pain after a disease (*e.g.,* atrophic vulvovaginitis associated with breast cancer) is established or prior to secondary exposure events (for example treat for a few weeks before attempting intercourse again).

As used herein, "inflammation" refers to the complex biological response to harmful stimuli, such as pathogens, damaged cells, or irritants. Inflammation is a protective attempt by the organism to remove the injurious stimuli as well as initiate the healing process for the tissue. Accordingly, the term "inflammation" includes any cellular process that leads to the production of pro-inflammatory cytokines, inflammation mediators and/or the related downstream cellular events resulting from the actions of the cytokines thus produced, for example, fever, fluid accumulation, swelling, abscess formation, and cell death. Pro-inflammatory cytokines and inflammation mediators include, but are not limited to, IL-1-alpha, IL-1-beta, IL-6, IL-8, IL-11, IL-12, IL-17, IL-18, TNF-alpha, leukocyte inhibitory factor (LIF), IFN-gamma, Oncostatin M (OSM), ciliary neurotrophic factor (CNTF), TGF-beta, granulocyte-macrophage colony stimulating factor (GM-CSF), and chemokines that chemoattract inflammatory cells. Inflammation can include both acute responses (*i.e.,* responses in which the inflammatory processes are active) and chronic responses (*i.e.,* responses marked by slow progression and formation of new connective tissue). Acute and chronic inflammation may be distinguished by the cell types involved. Acute inflammation often involves polymorphonuclear neutrophils; whereas chronic inflammation is normally characterized by a lymphohistiocytic and/or granulomatous response.

As disclosed herein, a number of ranges of values are provided. It is understood that each intervening value, to the tenth of the unit of the lower limit, unless the context clearly dictates otherwise, between the upper and lower limits of that range is also specifically disclosed. Each smaller range between any stated value or intervening value in a stated range and any other stated or intervening value in that stated range is encompassed within the invention. The upper and lower limits of these smaller ranges may independently be included or excluded in the range, and each range where either, neither, or both limits are included in the smaller ranges is also encompassed within the invention, subject to any specifically excluded limit in the stated range. Where the stated range includes one or both of the limits, ranges excluding either or both of those included limits are also included in the invention.

The term "about" or "approximately" means within an acceptable range for the particular value as determined by one of ordinary skill in the art, which will depend in part on how the value is measured or determined, *e.g.,* the limitations of the measurement system. For example, "about" can mean a range of up to 20%, preferably up to 10%, more preferably up to 5%, and more preferably still up to 1% of a given value. Alternatively, particularly with respect to biological systems or processes, the term can mean within an order of magnitude, preferably within 5-fold, and more preferably within 2-fold, of a value. Unless otherwise stated, the term 'about' means within an acceptable error range for the particular value.

### EXAMPLES

### Example 1

In this example, a fibroblast-based *in vitro* LPV model was established. Briefly, fibroblast strains were obtained from two regions (FIG. 1) of the lower genital tract of localized provoked vulvodynia (LPV) cases and pain-free controls in the manner described in Falsetta et al. Am J Obstet Gynecol 2015, vol. 213, pp. 38 e1-12 and Foster et al., Pain 2015, vol. 156, pp. 386-96.

Over 30 paired fibroblast strains obtained from LPV-afflicted cases (fulfilling Friedrich's Criteria)²⁵ and age/race-matched (critical variables influencing LPV),^{26, 27} pain-free controls were obtained. All subjects who contributed to the library were premenopausal, denied oral corticosteroids, non-steroidal, immunomodulatory, or anti-inflammatory medication use, and reported an absence of systemic/cutaneous inflammatory conditions at the time of sample collection. Mechanical pain thresholds were determined (via a method of limits) (0.5-5 N) using a Wagner algometer. Fibroblast identity was confirmed by microscopy and fibroblast markers (vimentin, collagen); only low passage (~4) cells are used.

The fibroblast strains were then challenged with stimuli such as *Candida ablicans* (FIG. 3A). It was found that fibroblasts taken from the painful vestibule of LPV patients produced high levels of IL-6 when infected with *Candida ablicans,* even at doses lower than those normally detectable within the vulvovaginal milieu, while fibroblasts from non-painful external vulva are weakly responsive **(****FIG. 3A****).** *C. albicans* is a chief cause of vulvovaginal yeast infection,¹⁹⁻²² and chronic yeast infection has been cited as a preceding factor in >70% LPV patients.²³ Repeated vulvovaginal infection in mice induces vulvar allodynia and regional hyperinnervation, simulating vulvodynia findings.²⁴ LPV is associated with inflammatory dysregulation, despite the fact LPV does not present as a classical inflammatory disease. The cardinal signs of inflammation are not pronounced or are vaguely present in both healthy and LPV-afflicted women, although the infiltration and organization of immune cells is distinctively different in LPV versus healthy patients.¹⁷

In addition, it was found that there was a site-specific response to live yeast infection, whereby fibroblasts from sites of pain within the vulvar vestibule are inherently sensitive to yeast/yeast products and produce elevated levels of pro-pain/ proinflammatory mediators compared to fibroblasts from non-painful sites of the external vulva (**FIG. 4B**). Furthermore, this response appeared to be an exacerbation of a normal inflammatory response, as fibroblasts from the vestibule of healthy women showed a similar, albeit reduced, response to zymosan (yeast cell wall product; **FIG. 4B**). More importantly, there was a strong connection between LPV pain and inflammation; pain in LPV patients was directly correlated with the production of proinflammatory and pro-pain mediators by fibroblasts cultured from biopsies of painful sites, when exposed to live yeast (**FIG. 4C**).

### Example 2

In this example, assays were carried out to investigate the ability of SPMs to reduce proinflammatory and pro-pain mediator production from primary human cells in the *in vitro* LPV model described in Example 1.

Synthesized, purified and commercially available SPMs from each known SPM class (E-series Resolvins, D-series Resolvins, Maresins, Protectins and Lipoxins) at low/nanomolar concentrations (1-100 nM) were tested. These concentrations have been shown to be effective in resolving inflammation in both *in vitro* and *in vivo* model systems without any toxicity. The inventors used one of two treatment regimens proven effective in vulvar fibroblasts and other cells: 1) overnight pre-treatment, followed by another treatment 30 min prior to stimulation with proinflammatory stimuli for 48 hr with a third dose of SPMs at 24 hr post-challenge, or 2) post-treatment with SPMs after a 30 min pre-treatment with inflammatory stimuli, followed by a booster dose 18 hr later. Both treatment regimens are of interest, as SPMs are active throughout the inflammatory process.⁵⁻⁷ Even SPMs administered after LPV onset are likely to prevent the worsening or spread of LPV pain.

The data show that out of 8 SPMs (from 3 classes) tested, at least 4 were highly effective in reducing proinflammatory mediators linked to pain in human vulvar fibroblasts treated with relevant proinflammatory stimuli using a pre-treatment strategy. Lipoxin A₄, Resolvin D₂, Maresin 1 and epi-Maresin 1 significantly reduced prostaglandin E₂ (PGE₂; **FIG. 5A**) and interleukin-6 (IL-6; **FIG. 5B**) production by both vestibular and external vulvar fibroblasts. However, Resolvin D₁, 17(S)-HDHA, Lipoxin B₄, and AT-Resolvin D₁ showed no striking effects in initial tests. Maresin 1 and Lipoxin A₄ were also tested using the post-treatment regimen and found both significantly reduced IL-6 and PGE₂ levels under this strategy (**FIGs. 5C** and **5D**).

Next, SPMs from other classes (E series Resolvins and Protectins), as well as additional SPMs from classes containing members that are effective in reducing IL-6 and PGE₂ production (e.g., Maresins) were investigated in similar screen. Live *C*. *albicans* yeast, zymosan, bradykinin, and IL-1β all can be used as different classes of inflammatory activators, which have been shown to induce the production of proinflammatory mediators in vulvar fibroblasts.^{3, 4, 13, 15} Proinflammatory mediator levels were measured using ELISA and EIA assays. SPMs that were effective in reducing more than one proinflammatory mediator in at least 2 tests can be examined for further testing using a preclinical mouse model. Congruent with the above supporting results showing several SPMs were effective in reducing proinflammatory mediator production, inventors identified several additional SPMs that are highly effective as a pre-treatment (FIGs. 23A and 23B) and post-treatment (FIGs. 24A and 24B). These additional effective SPMs include Resolvin D₃, Resolvin D₄, Resolvin D₅, Resolvin E₁, Protectin D1, and Protectin DX. SPMs meeting criteria for further testing can be tested for their ability to reduce pain and inflammatory endpoints in a mouse model of LPV as shown in the examples below.

Prior to this work, no therapeutic agents effective in reducing the proinflammatory/pro-pain mediators associated with LPV had been identified. Therefore, this work represents a significant step forward in identifying potential therapeutic agents that could not only reduce excessive proinflammatory signaling in the context of LPV, but also in other chronic inflammatory conditions.

### Example 3

In this example a number of SPMs are tested for their ability to reduce pain and inflammatory endpoints in a mouse model of LPV. Only recently has an initial animal model of LPV been developed.²⁴ This original model has not been used for the preclinical testing of therapeutic agents. Preclinical testing is an essential step in the development of new effective FDA-approved therapies, which are sorely needed for LPV.^{28, 29} Therefore, there is an urgent unmet need for a preclinical animal model that accurately reflects human LPV that could be used prior to human clinical trials. Congruent with this need, this optimized mouse model of LPV is ideal for the task of preclinical testing of therapeutic agents, as evidenced by strong supporting data.

Although SPM structure and therapeutic efficacy is conserved across species,⁵⁻⁷ inventors first confirmed that mouse vulvar tissues responded to SPM treatment *in vitro* by culturing mouse vulvar explants (4 mm punch biopsy). The explants were stimulated with IL-1β, and then assays were carried to assess the ability of Maresin 1 or RvD₂ to reduce PGE₂ production under the established pre-treatment regimen.

It was found that enhanced PGE₂ responses following IL-1β treatment were significantly suppressed with Maresin 1 or RvD₂ over a range of doses, as low as 1 nM (**FIG. 6**). These results confirmed that the mouse vulva responded to SPM treatment, akin to human fibroblasts. It was also determined that these mice expressed several SPM receptors (*e.g.,* ALX, GPR18). Therefore, inventors proceeded with testing responses to SPMs in the mouse model.

In the mouse model, zymosan (a proinflammatory yeast cell wall preparation) was used to induce sustained vulvar allodynia, measured by pain threshold testing. Inventors initially used a manual von Frey system (MvF) ²⁴, but later switched to an electronic system as detailed herein. MvF employed a series of "hairs" of different thicknesses/rigidity that exert differing forces when applied to the injection site, located at the midline posterior vulvar (between the vaginal opening and anus) (**FIG. 7A**). The hair was applied perpendicular to the vulvar surface with a gradually increasing force within a range of 0.100 g to 4.0 g (**FIG. 7B**). A positive response was defined as either a clear reflexive, all 4 extremity extension, jump, or immediate grooming of the vulva in response to vulvar stimulus. To determine the MvF threshold, the "up down method" was followed.³⁰ During allodynia induction, the mice receive weekly injections of zymosan (10 µg/ml in 10 µl saline) for a maximum of 6 injections, until a >33% reduction in pain threshold is observed for two consecutive weeks of testing (**FIG. 7C**). Pain threshold testing was performed at the same time every week, immediately prior to zymosan injection; after the first two weeks of injections, a determination of threshold change was performed after pain testing to determine which mice would receive additional zymosan injections. Saline injections, which contain no proinflammatory agent, served as the negative control.

Using this approach, inventors confirmed that one could induce vulvar allodynia, measure pain responses via mechanical threshold determination, and assess treatment responses. However, inventors implemented several modifications to improve the robustness of the model including the following: **1)** use a genetically tractable inbred strain, **2)** validation of the use of an electronic von Frey (EvF) system, **3)** assessment of the impact of behavioral conditioning on pain response, **4)** weekly collection of vulvovaginal lavages for proinflammatory mediator quantification, and **5)** testing the ability of a selected SPM (*e.g.*, Maresin 1) to modulate pain and inflammation in our new model. These improvements overcome two significant drawbacks in the original model: **1)** MvF determinations of threshold are time-consuming and only one quantitative value is generated per test, and **2)** the use of an outbred strain prevents genetic manipulation and increases study variability (original model strain is outbred CD-1). Thus, inventors elected to test two commonly used, genetically tractable mouse strains: C57BL/6 and BALB/c. Inventors had the greatest success in the C57BL/6 background and test key SPMs identified in Example 2 in C57BL/6 mice (*e.g.*, Maresin) as potential pain/inflammation resolving agents. Using the same experimental setup as pilot studies **(**FIG. **7C****),** but replacing MvF measurement with a Mousemet EvF system (Topcat Metrology) and implementing the other aforementioned modifications, it was found that weekly pain tests generated consistent reproducible values (average of 5 trials/mouse) for each C57BL/6 mouse (**FIG. 8**). Collectively, inventors saw a progressive lowering of pain thresholds in mice receiving zymosan, which was accompanied by an increase in vaginal PGE₂ (**FIG. 8**). It was confirmed that allodynia persisted for a period of at least 5 weeks after allodynia induction (**FIG. 8**) before initiating daily treatments with 1 µg/mouse/day Maresin 1 for a period of 4 weeks. With treatment, inventors saw a complete restoration to the pre-induction thresholds, while PGE₂ levels were suppressed and maintained at levels below baseline (**FIG. 9**)**.** These impressive results suggest that one or more SPMs are effective.

### Example 4

In this example, assays were carried out to investigate the ability of DHA, a SPM precursor, to reduce pain and inflammatory endpoints using a mouse model of LPV as described in Example 3.

A total of 36 C57BL/6J mice were subjected to 6 weeks of allodynia induction using the protocol described above. Briefly, zymosan was used to induce sustained vulvar allodynia, measured by pain threshold testing. An electronic vonFrey system (MOUSEMET^{™}, Topcat Metrology) was used to apply gradually increasing force to the vulva at the injection site, located at the midline posterior vulvar (between the vaginal opening and anus) (**FIG. 7A**). The hair was applied perpendicular to the vulvar surface with a gradually increasing force within a range of 0.100 g to 7.0 g (**FIG. 7B**). A positive response was defined as either **(1)** a reflexive, coordinated four extremity extension, **(2)** jump, or **(3)** immediate grooming of the vulva in response to vulvar stimulus, at which point the peak force was recorded.

During allodynia induction, mice receive weekly injections of zymosan (10 mg/ml in 10 µl saline) for a maximum of 6 injections, until a >33% reduction in pain threshold is observed for 2 consecutive weeks of testing (**FIG. 7C**). Pain threshold testing was performed at the same time every week, immediately prior to zymosan injection; after the first 2 weeks of injections, a determination of threshold change was performed after pain testing to determine which mice would receive additional zymosan injections.

After 6 weeks of induction, a total of 35 mice developed allodynia, which were divided into the following treatment groups: DHA cream (12 mice), placebo cream (12 mice), and mock treatment (11 mice). DHA and placebo cream mice received twice daily topical application of the designated cream via a cotton swab, Monday through Friday and single daily application on Saturdays and Sundays for a total of 6 weeks. The entire shaved area was coated with a thin layer of cream prior to release into the home cage. Mice receiving mock treatment were treated for the same length of time over the same vulvar area with a cotton swab moistened with sterile PBS.

Mice were determined to have "recovered" from allodynia when they showed a 70% improvement over their lowest pain threshold (at the end of the 6-week allodynia induction phase) for 2 consecutive pain tests. After 6 weeks of treatment, 9 mice had recovered in each the DHA and placebo groups, while 6 mice had recovered in the mock treatment group (**FIG. 13**). The same results were obtained when "recovery" was defined as a threshold that was at least 70% of the pre-pain threshold, prior to zymosan injection. Thus, there were no differences in the number of mice that recovered in the DHA versus the placebo group, at the conclusion of treatment. However, when looking at recovery over time, inventors saw temporal treatment differences between DHA and placebo cream (**FIG. 13**). Mice in the DHA cream group began to recover as early as the first two weeks of treatment, while only one mouse had recovered in the placebo cream group; it took 3-4 weeks for the placebo cream group to catch up to the DHA cream group. Overall, fewer mice recovered in the mock group, but we began to see evidence of recovery around 4 weeks, which may be reflective of natural pain resolution in the model.

Correlating with these results, it was found that the percent improvement scores were higher in the DHA group for the first 2 weeks, while there were no differences in the percent improvement scores at week 3 (**FIG. 14**). The percent improvement scores for the mock mice tended to be lower than the scores in either the DHA or placebo groups.

Inventors established a link between pain thresholds and vulvovaginal PGE₂ levels in C57BL/6J mice. Greater vaginal PGE₂ levels predict greater pain sensitivity. Such findings are in agreement with results from human studies. PGE₂ levels produced by cultured primary human vulvar fibroblast strains can predict the pain threshold at the site from which the fibroblasts were collected by biopsy. Inventors conducted weekly vulvovaginal lavages on live mice throughout the induction and treatment phases and found that levels of PGE₂ were initially low prior to zymosan induction, but rose sharply with zymosan injection, reaching a peak at week 4 of the injection series (**FIG. 15**). These levels were sustained prior to treatment, but then dropped rapidly and remained low in the placebo and DHA groups. However, there were no significant differences in PGE₂ levels between the DHA and placebo groups. It was also found that IL-6 levels were low or undetectable in most vulvovaginal lavages, consistent with prior experiments.

Overall, DHA and placebo (cream only) both improved vulvar pain outcomes to a similar extent after the full 6-week course of treatment. However, DHA improved pain outcomes more rapidly than placebo, and both outperformed mock treatment. The placebo cream does contain long chain alcohols and fatty acids that may have soothing or humectant properties. Therefore, improvement in the placebo group is not entirely surprising. Reducing the concentrations of these compounds in future trials could help to enhance the detection of DHA-specific effects. These results suggest that the DHA cream is effective in reducing human vulvar pain.

### Example 5

In this example, assays were carried out to investigate the ability of a mixture containing docosahexaenoic acid (DHA) and additional specialized pro-resolving mediator (SPM) precursor molecules (14-HDHA, 17-HDHA, and 18-HEPE) to reduce pain and inflammatory endpoints using a mouse model of LPV as described above.

A total of 52 C57BL/6J mice were subjected to 4 weeks of allodynia induction using the above described protocol. In brief, zymosan (a proinflammatory yeast cell wall preparation) was used to induce sustained vulvar allodynia, measured by pain threshold testing. An electronic vonFrey system (Mousemet, Topcat Metrology) was used to apply gradually increasing force to the vulva at the injection site, located at the midline posterior vulvar (between the vaginal opening and anus) (**FIG. 7A**). The hair was applied perpendicular to the vulvar surface with a gradually increasing force within a range of 0.100 g to 7.0 g (**FIG. 7B**). A positive response was defined as either **(1)** a clear reflexive, all 4 extremity extension, **(2)** jump, or **(2)** immediate grooming of the vulva in response to vulvar stimulus, at which point the peak force is recorded. During allodynia induction, mice receive weekly injections of zymosan (10 mg/ml in 10 µl saline) for a total of 4 injections. Weekly thresholds were determined, but only after the 4^{th} injection was a decision made as to which mice continue to phase 2 (drug testing). Mice that exhibited a >33% reduction in pain threshold (+/- 0.5 g force) for 2 consecutive weeks of testing moved on to phase 2. Pain threshold testing was performed at the same time every week, immediately prior to zymosan injection. After the 4^{th} injection, the mice underwent a final round of threshold testing (on week 5) to determine if they met the criteria to enter into phase 2.

After 4 weeks of induction, a total of 48 mice developed allodynia, which were divided into the following treatment groups: LIPINOVA cream high dose (1.9% LIPINOVA by volume; 12 mice), LIPINOVA low dose (0.7% LIPINOVA by volume; 12 mice), placebo (base cream without LIPINOVA; 11 mice), and mock treatment (saline moistened swab; 11 mice). LIPINOVA is a highly purified fish oil product that contains -40% docosahexaenoic acid (DHA) by volume with additional specialized pro-resolving mediator (SPM) precursor molecules (14-HDHA, 17-HDHA, and 18-HEPE). Mice received twice daily topical application of the designated cream Monday through Friday and single daily application on Saturdays and Sundays for a total of 4 weeks. Mice received an application of cream via a cotton swab for ~15 sec. The entire shaved area was coated with a thin layer of cream prior to release into the home cage. Mice receiving mock treatment were treated for the same length of time with a cotton swab moistened with sterile PBS. Mice were determined to have "recovered" from allodynia when their threshold returned to 66% of the pre-pain induction baseline (+/- 0.5 g force) for 2 consecutive pain tests. After 4 weeks of treatment, 8 mice in the LIPINOVA high dose group had recovered (67%), while only 2 mice in the low dose group had recovered (17%) (**FIG. 16**). No mice in the mock treatment group recovered within the 4-week period, while a single mouse recovered in the placebo group (9%). Mice in the LIPINOVA high dose group showed steady recovery with several additional mice recovering each week, while with the low dose, a couple mice recovered early (week 2), but no additional mice recovered by week 4. These data suggest that recovery was influenced by LIPINOVA dose, with higher doses being more effective, while placebo and mock treatments were largely ineffective. There was a single mouse that recovered with placebo, which could likely be attributed to the humectants in the base cream.

Additional measures were used to evaluate the effects of treatment, such as the percent improvement with time **(**FIGs **17A and 17B**). The percent improvement was calculated as the percent increase in weekly threshold values over the last pre-treatment pain threshold (final week of phase 1). In **FIG. 17A****,** the data was graphed as the average percent recovery for all mice within the group (+/- SEM). The percent recovery for each mouse was determined and then the values for the entire group were averaged. The mice in the LIPINOVA high dose treatment group showed consistently higher percent recovery scores each week versus the other groups. There was little difference in the score for the LIPINOVA low dose compared to controls (mock and placebo), except during week 3, where the percent recovery score for the LIPINOVA low group was closer to the LIPINOVA high group than the controls. In **FIG. 17B****,** the same data was graphed as a box and whisker plot, where each dot represents the value for an individual mouse. Again, this data showed that the percent recovery scores where higher on average in the LIPINOVA high group. This graph shows that at week 4, the mock group median, which is similar to the LIPINOVA high median is being skewed by a couple mice with particularly high percent improvement scores that week only. Overall, this data demonstrates that treatment with the higher dose of LIPINOVA is most effective in improving pain threshold scores.

The pain threshold values were also graphed as the percent of the original baseline prior to pain induction in phase 1 (**FIGs. 18A** and **18B**). Individual percent baseline values were used to determine recovery for each mouse (**FIG. 16**). In **FIG. 18A****,** these values were graphed as the average for each group (+/- SEM). Again, this data showed the LIPINOVA high dose consistently outperformed the other treatments, including the LIPINOVA low dose. By week 4, only the LIPINOVA high group had crossed the 66% percent recovery threshold for the group, which could be attributed to observation that the majority of the mice had recovered by week 4 (67%). In the box and whisker plot in **FIG. 18B****,** it is apparent that the percent baseline score is consistently higher for the mice in the LIPINOVA high group. At week 4, the mock group median approached the LIPINOVA high group median, but again, this could likely be attributed to a couple mice with particularly high threshold scores that week.

### Example 6

In this example, the mice tested in Examle 5 above were furhter examined for the effect of LIPINOVA to reduce pain and inflammatory endpoints in the manner described above for a period of 17 weeks.

A total of 46 mice that developed allodynia were divided into the following treatment groups: LIPINOVA high dose (1.9%) cream (12 mice), LIPINOVA low dose (0.7%) cream (12 mice), placebo cream (11 mice), and mock treatment (11 mice). LIPINOVA and placebo-treated mice received twice daily topical application of the designated cream via a cotton swab, Monday through Friday and single daily application on Saturdays and Sundays for a total of 6 weeks. The entire shaved area was coated with a thin layer of cream prior to release into the home cage. Mice receiving mock treatment were treated for the same length of time over the same vulvar area with a cotton swab moistened with sterile PBS.

After 6 weeks of treatment, treatment was withdrawn and mice were monitored for another 7 weeks to determine if recovery was sustained. At the end of those 7 weeks, mice in the placebo group resumed placebo treatment and mice in the mock group received a combination therapy of 1.9% LIPINOVA with 1% pramoxine. The mice continued for another 3 weeks, at which point the trial was discontinued because a majority of the mice had naturally recovered.

Mice were determined to have "recovered" from allodynia when they returned to at least 70% of their pre-pain baseline threshold for 2 consecutive pain tests. After 6 weeks of treatment, 11 mice had recovered in the LIPINOVA high dose group, while only 2 mice had recovered in each other group (**FIG. 19**). The same results were obtained when we defined "recovery" as at least a 70% improvement over the lowest pain threshold (last pain threshold prior to treatment).

When the longevity of treatment effects in Phase 3 were assessed (**FIG. 19**), the inventors found that the effects of treatment were sustained greater than 7 weeks in the LIPINOVA high group, while the inventors continued to see additional recovery in the low dose, mock, and placebo groups. Of note, the LIPINOVA low group showed enhanced recovery compared to the mock and placebo groups, suggesting low dose LIPINOVA may help boost natural recovery, although mice do not recover as quickly and completely as mice receiving the high dose.

The inventors also attempted to add a fourth phase to test an additional therapy (combination of LIPINOVA high dose and 1% pramoxine). Unfortunately, mice began to recover naturally at this time. Ultimately, the inventors found that allodynia could be sustained in mice for a period of about 20 weeks, giving an adequate window to test both therapeutic application and the effects of treatment withdrawal.

Correlating with these results, the inventors found the percent baseline scores were higher in the LIPINOVA high dose group than any other group during the treatment period and beyond (**FIG. 20**). During treatment, mock and placebo group scores were similar, indicating no strong effect from the placebo.

The inventors found very similar results when they evaluated the data using percent improvement scores (**FIG. 21**), which is another way to examine the same threshold data. This measure looks at the improvement over the lowest scores rather than the relevance to the starting point (before pain induction). This metric is likely more meaningful clinically, as one cannot necessarily determine what the patient's baseline was, while he or she can evaluate their current pain.

The inventors established a link between pain thresholds and vulvovaginal PGE₂ levels in C57BL/6J mice Greater vaginal PGE₂ levels predict greater pain sensitivity. Such findings were in agreement with results from human studies. PGE₂ levels produced by cultured primary human vulvar fibroblast strains can predict the pain threshold at the site from which the fibroblasts were collected by biopsy. The inventors conducted weekly vulvovaginal lavages on live mice throughout the induction and treatment periods and found that levels of PGE₂ were initially low prior to zymosan induction, but rose sharply with zymosan injection, reaching a peak at week 3 of the injection series (**FIG. 22**). These levels then waned over time with treatment. However, there were no significant differences in PGE₂ levels between the treatment groups, despite observing these levels tended to be lower in the LIPINOVA high dose group.

Overall, LIPINOVA, at the higher 1.9% dose, can improve vulvar pain outcomes and restore mice to pre-pain thresholds in less than 6 weeks. However, there is no strong correlation between treatment and reductions in vulvovaginal levels of PGE₂. There is a trend suggesting levels may be lower with high dose treatment, but it is not statistically significant. There was no significant improvement in the control groups or the LIPINOVA low dose during the treatment phase; only 2 mice (<20%) recovered in each of these groups. However, the LIPINOVA low dose group recovered faster than the placebo and mock groups during the withdrawal phase. Altogether, these results suggest LIPINOVA is effective in promoting the resolution of inflammation and reducing pain in a mouse model of vulvodynia.

In sum, high dose LIPINOVA (1.9%) dramatically reduced zymosan-induced vulvar pain in mice receiving topical application twice daily Monday-Friday and once daily Saturday and Sunday. This treatment was more effective than low dose LIPINOVA (0.7%) or controls (placebo and mock). Nearly all the mice receiving 1.9% LIPINOVA (11/12, 92%) recovered by five weeks of treatment compared to <17% recovering in any other group at the same time point. Over time, mice in the low dose LIPINOVA group (0.7%) showed enhanced recovery compared to placebo or mock treated mice, especially during the withdrawal period. The effects of LIPINOVA treatment were sustained for more than 8 weeks after treatment withdrawal. PGE₂ levels were inversely correlated with vulvar pain threshold; higher PGE₂ levels corresponded to lower pain thresholds and predicted greater pain sensitivity. With treatment, PGE₂ levels decreased over time as thresholds rebounded and pain sensitivity decreased. However, no significant differences were found between treatment groups, despite slightly lower values in the 1.9% LIPINOVA group. These results suggest that the LIPINOVA cream will be highly effective in reducing human vulvar pain.

### References

1. Sadownik, LA. Etiology, diagnosis, and clinical management of vulvodynia. Int J Womens Health 2014, vol. 6, pp. 437-49, PMID: 24833921, PMCID: PMC4014358.
2. Haefner, HK; Collins, ME; Davis, GD; Edwards, L; Foster, DC; Hartmann, ED; Kaufman, RH; Lynch, PJ; Margesson, LJ; Moyal-Barracco, M; Piper, CK; Reed, BD; Stewart, EG; Wilkinson, EJ. The vulvodynia guideline. Journal of Lower Genital Tract Disease 2005, vol. 9, pp. 40-51, PMID: 15870521, PMCID:
3. Falsetta, MI,; Foster, DC; Woeller, CF; Pollock, SJ; Bonham, AD; Haidaris, CG; Stodgell, CJ; Phipps, RP. Identification of novel mechanisms involved in generating localized vulvodynia pain. Am J Obstet Gynecol 2015, vol. 213, pp. 38 e1-12, PMID: 25683963, PMCID: PMC4485605.
4. Foster, DC; Falsetta, MI,; Woeller, CF; Pollock, SJ; Song, K; Bonham, A; Haidaris, CG; Stodgell, CJ; Messing, SP; Iadarola, M; Phipps, RP. Site-specific mesenchymal control of inflammatory pain to yeast challenge in vulvodynia-afflicted and pain-free women. Pain 2015, vol. 156, pp. 386-96, PMID: 25679469, PMCID: PMC4378529.
5. Buckley, CD; Gilroy, DW; Serhan, CN; Stockinger, B; Tak, PP. The resolution of inflammation. Nat Rev Immunol 2013, vol. 13, pp. 59-66, PMID: 23197111, PMCID:
6. Serhan, CN; Chiang, N; Dalli, J. The resolution code of acute inflammation: Novel pro-resolving lipid mediators in resolution. Semin Immunol 2015, vol. 27, pp. 200-15, PMID: 25857211, PMCID: PMC4515371.
7. Serhan, CN; Chiang, N; Dalli, J; Levy, BD. Lipid mediators in the resolution of inflammation. Cold Spring Harb Perspect Biol 2015, vol. 7, p a016311, PMID: 25359497, PMCID:
8. Harlow, BL; Kunitz, CG; Nguyen, RH; Rydell, SA; Turner, RM; MacLehose, RF. Prevalence of symptoms consistent with a diagnosis of vulvodynia: population-based estimates from 2 geographic regions. Am J Obstet Gynecol 2014, vol. 210, pp. 40 e1-8, PMID: 24080300, PMCID: PMC3885163.
9. Cui, JG; Holmin, S; Mathiesen, T; Meyerson, BA; Linderoth, B. Possible role of inflammatory mediators in tactile hypersensitivity in rat models of mononeuropathy. Pain 2000, vol. 88, pp. 239-48, PMID: 11068111, PMCID:
10. Lin, CR; Amaya, F; Barrett, L; Wang, H; Takada, J; Samad, TA; Woolf, CJ. Prostaglandin E2 receptor EP4 contributes to inflammatory pain hypersensitivity. Journal of Pharmacology and Experimental Therapeutics 2006, vol. 319, pp. 1096-103, PMID: 16966471, PMCID:
11. Akopians, AL; Rapkin, AJ. Vulvodynia: The Role of Inflammation in the Etiology of Localized Provoked Pain of the Vulvar Vestibule (Vestibulodynia). Semin Reprod Med 2015, vol. 33, pp. 239-45, PMID: 26132928, PMCID:
12. Baker, DA; Peresleni, T; Kocis, C. Inflammatory Markers in Vestibulodynia [4]. Obstet Gynecol 2016, vol. 127 Suppl 1, pp. 1S-2S, PMID: 27176158, PMCID:
13. Falsetta, MI,; Foster, DC; Woeller, CF; Pollock, SJ; Bonham, AD; Haidaris, CG; Phipps, RP. A role for bradykinin signaling in chronic vulvar pain. J Pain 2016, PMID: 27544818, PMCID:
14. Leclair, N; Thormer, G; Sorge, I; Ritter, L; Schuster, V; Hirsch, FW. Whole-Body Diffusion-Weighted Imaging in Chronic Recurrent Multifocal Osteomyelitis in Children. PLoS One 2016, vol. 11, p e0147523, PMID: 26799970, PMCID: PMC4723072.
15. Foster, DC; Piekarz, KH; Murant, TI; LaPoint, R; Haidaris, CG; Phipps, RP. Enhanced synthesis of proinflammatory cytokines by vulvar vestibular fibroblasts: implications for vulvar vestibulitis. Am J Obstet Gynecol 2007, vol. 196, pp. 346 e1-8, PMID: 17403416, PMCID:
16. Morgan, TK; Allen-Brady, KL; Monson, MA; Leclair, CM; Sharp, HT; Cannon-Albright, LA. Familiality analysis of provoked vestibulodynia treated by vestibulectomy supports genetic predisposition. Am J Obstet Gynecol 2016, vol. 214, pp. 609 e1-7, PMID: 26627726, PMCID:
17. Tommola, P; Butzow, R; Unkila-Kallio, L; Paavonen, J; Meri, S. Activation of vestibule-associated lymphoid tissue in localized provoked vulvodynia. Am J Obstet Gynecol 2015, vol. 212, pp. 476 e1-8, PMID: 25448516, PMCID:
18. Tommola, P; Unkila-Kallio, L; Paetau, A; Meri, S; Kalso, E; Paavonen, J. Immune activation enhances epithelial nerve growth in provoked vestibulodynia. Am J Obstet Gynecol 2016, PMID: 27457118, PMCID:
19. Cassone, A. Vulvovaginal Candida albicans infections: pathogenesis, immunity and vaccine prospects. BJOG 2015, vol. 122, pp. 785-94, PMID: 25052208, PMCID:
20. Davies, S; Johnson, E; White, D. How to treat persistent vaginal yeast infection due to species other than Candida albicans. Sex Transm Infect 2013, vol. 89, pp. 165-6, PMID: 23180861, PMCID:
21. Giraldo, P; von Nowaskonski, A; Gomes, FA; Linhares, I; Neves, NA; Witkin, SS. Vaginal colonization by Candida in asymptomatic women with and without a history of recurrent vulvovaginal candidiasis. Obstet Gynecol 2000, vol. 95, pp. 413-6, PMID: 10711554, PMCID:
22. Harriott, MM; Lilly, EA; Rodriguez, TE; Fidel, PL, Jr.; Noverr, MC. Candida albicans forms biofilms on the vaginal mucosa. Microbiology 2010, vol. 156, pp. 3635-44, PMID: 20705667, PMCID: 3068702.
23. Donders, G; Bellen, G. Characteristics of the pain observed in the focal vulvodynia syndrome (VVS). MedHypotheses 2012, vol. 78, pp. 11-4, PMID: 22041052, PMCID:
24. Farmer, MA; Taylor, AM; Bailey, AL; Tuttle, AH; MacIntyre, LC; Milagrosa, ZE; Crissman, HP; Bennett, GJ; Ribeiro-da-Silva, A; Binik, YM; Mogil, JS. Repeated vulvovaginal fungal infections cause persistent pain in a mouse model of vulvodynia. Sci Transl Med 2011, vol. 3, p 101ra91, PMID: 21937756, PMCID: PMC3243907.
25. Bergeron, S; Binik, YM; Khalife, S; Pagidas, K; Glazer, HI. Vulvar vestibulitis syndrome: reliability of diagnosis and evaluation of current diagnostic criteria. Obstet Gynecol 2001, vol. 98, pp. 45-51, PMID: 11430955, PMCID:
26. Edwards, RR; Doleys, DM; Fillingim, RB; Lowery, D. Ethnic differences in pain tolerance: clinical implications in a chronic pain population. Psychosom Med 2001, vol. 63, pp. 316-23, PMID: 11292281, PMCID:
27. Wolf, J; Weinberger, B; Arnold, CR; Maier, AB; Westendorp, RG; Grubeck-Loebenstein, B. The effect of chronological age on the inflammatory response of human fibroblasts. Exp Gerontol 2012, vol. 47, pp. 749-53, PMID: 22790019, PMCID: PMC3427851.
28. Brown, CS; Foster, DC; Wan, JY; Rawlinson, LA; Bachmann, GA; Gabapentin Study, G. Rationale and design of a multicenter randomized clinical trial of extended release gabapentin in provoked vestibulodynia and biological correlates of response. Contemp Clin Trials 2013, vol. 36, pp. 154-65, PMID: 23816491, PMCID: PMC3779071.
29. Foster, DC; Kotok, MB; Huang, LS; Watts, A; Oakes, D; Howard, FM; Poleshuck, EL; Stodgell, CJ; Dworkin, RH. Oral desipramine and topical lidocaine for vulvodynia: a randomized controlled trial. Obstetrics and Gynecology 2010, vol. 116, pp. 583-93, PMID: 20733439, PMCID:
30. Chaplan, SR; Bach, FW; Pogrel, JW; Chung, JM; Yaksh, TL. Quantitative assessment of tactile allodynia in the rat paw. J Neurosci Methods 1994, vol. 53, pp. 55-63, PMID: 7990513, PMCID:
31. Baglole, CJ; Maggirwar, SB; Gasiewicz, TA; Thatcher, TH; Phipps, RP; Sime, PJ. The aryl hydrocarbon receptor attenuates tobacco smoke-induced cyclooxygenase-2 and prostaglandin production in lung fibroblasts through regulation of the NF-kappaB family member RelB. J Biol Chem 2008, vol. 283, pp. 28944-57, PMID: 18697742, PMCID: 2_5708_56
32. Colucci, M; Maione, F; Bonito, MC; Piscopo, A; Di Giannuario, A; Pieretti, S. New insights of dimethyl sulphoxide effects (DMSO) on experimental in vivo models of nociception and inflammation. Pharmacol Res 2008, vol. 57, pp. 419-25, PMID: 18508278, PMCID:
33. Galer, BS. A comparative subjective assessment study of PENNSAID(R) and Voltaren Gel(R), two topical formulations of diclofenac sodium. Pain Pract 2011, vol. 11, pp. 252-60, PMID: 20854305, PMCID:
34. Xie, Y; Shi, L; Xiong, X; Wu, E; Veasley, C; Dade, C. Economic burden and quality of life of vulvodynia in the United States. Curr Med Res Opin 2012, vol. 28, pp. 601-8, PMID: 22356119, PMCID:
   The foregoing examples and description of the preferred embodiments should be taken as illustrating, rather than as limiting the present invention as defined by the claims.

## Claims

1. A pro-resolving mediator for use in a method of reducing or preventing lower genital tract irritation in a female subject, the method comprising administering an effective amount of the pro-resolving mediator topically to a treatment site of the subject's lower genital tract,
wherein the irritation is associated with a genital tract inflammatory condition selected from the group consisting of localized provoked vulvodynia (LPV), lichen planus, lichen sclerosus, desquamative inflammatory vaginitis, atrophic vulvovaginitis associated with breast cancer, and chronic pruritus, and
wherein the pro-resolving mediator is (1) selected from the group consisting of Resolvin D₂, Resolvin D₃, Resolvin D₄, Resolvin D₅, Resolvin E₁, Maresin-1, epi-Maresin-1, Lipoxin A₄, Protectin D1, Protectin DX, and DHA; or (2) a mixture comprising DHA, 14-HDHA, 17-HDHA, and 18-HEPE.

2. The pro-resolving mediator for use of claim 1, wherein the pro-resolving mediator is Maresin-1.

3. The pro-resolving mediator for use of claim 1, wherein the pro-resolving mediator is Protectin D1.

4. The pro-resolving mediator for use of any one of claims 1 to 3, wherein the genital tract inflammatory condition is LPV.

5. The pro-resolving mediator for use of any one of claims 1 to 4, wherein the pro-resolving mediator is administered at 0.0001 mg/kg - 100 mg/kg.

6. The pro-resolving mediator for use of any one of claims 1 to 5, wherein the pro-resolving mediator is administered once a week, 2-3 times a week, once a day, twice a day, or three times a day.

7. The pro-resolving mediator for use of any one of claims 1 to 6, wherein the pro-resolving mediator is administered before the subject is exposed to a secondary irritation-causing stimulation.

8. The pro-resolving mediator for use of any one of claims 1 to 7, wherein the treatment site comprises the vulvar vestibule.

9. The pro-resolving mediator for use of any one of claims 1 to 8, wherein the treatment site comprises the external vulva, vestibule, or vagina.

10. The pro-resolving mediator for use of claim 9, wherein the treatment site comprises the external vulva.

11. The pro-resolving mediator for use of any one of claims 1-10, further comprising administering a second therapeutic agent to the subject.

12. The pro-resolving mediator for use of claim 11, wherein the second therapeutic agent is a second pro-resolving mediator.

13. The pro-resolving mediator for use of claim 11, wherein the second therapeutic agent is an anti-microbial agent or an antiviral agent.

## Patentansprüche

1. Pro-auflösender Mediator zur Verwendung in einem Verfahren zum Verringern oder Verhindern von Reizungen des unteren Genitaltrakts bei einem weiblichen Subjekt, das Verfahren umfassend ein Verabreichen einer wirksamen Menge des proauflösenden Mediators topisch an eine Behandlungsstelle des unteren Genitaltrakts des Subjekts,
wobei die Reizung mit einer entzündlichen Erkrankung des Genitaltrakts assoziiert ist, ausgewählt aus der Gruppe, bestehend aus lokalisierter provozierter Vulvodynie (LPV), Lichen planus, Lichen sclerosus, desquamativer entzündlicher Vaginitis, atrophischer Vulvovaginitis assoziiert mit Brustkrebs, und chronischem Juckreiz, und
wobei der pro-auflösende Mediator (1) ausgewählt ist aus der Gruppe, bestehend aus Resolvin D₂, Resolvin D₃, Resolvin D₄, Resolvin D₅, Resolvin E₁, Maresin-1, epi-Maresin-1, Lipoxin A₄, Protectin D1, Protectin DX und DHA; oder (2) einem Gemisch, umfassend DHA, 14-HDHA, 17-HDHA und 18-HEPE.

2. Pro-auflösender Mediator zur Verwendung nach Anspruch 1, wobei der Pro-auflösende Mediator Maresin-1 ist.

3. Pro-auflösender Mediator zur Verwendung nach Anspruch 1, wobei der Pro-auflösende Mediator Protectin D1 ist.

4. Pro-auflösender Mediator zur Verwendung nach einem der Ansprüche 1 bis 3, wobei der entzündliche Zustand des Genitaltrakts LPV ist.

5. Pro-auflösender Mediator zur Verwendung nach einem der Ansprüche 1 bis 4, wobei der Pro-auflösende Mediator mit 0,0001 mg/kg - 100 mg/kg verabreicht wird.

6. Pro-auflösender Mediator zur Verwendung nach einem der Ansprüche 1 bis 5, wobei der Pro-auflösende Mediator einmal pro Woche, 2-3 mal pro Woche, einmal pro Tag, zweimal pro Tag oder dreimal pro Tag verabreicht wird.

7. Pro-auflösender Mediator zur Verwendung nach einem der Ansprüche 1 bis 6, wobei der Pro-auflösende Mediator verabreicht wird, bevor das Subjekt einer sekundären reizverursachenden Stimulation ausgesetzt wird.

8. Pro-auflösender Mediator zur Verwendung nach einem der Ansprüche 1 bis 7, wobei die Behandlungsstelle das Vulva-Vestibulum umfasst.

9. Pro-auflösender Mediator zur Verwendung nach einem der Ansprüche 1 bis 8, wobei die Behandlungsstelle die externe Vulva, das Vestibül oder die Vagina umfasst.

10. Pro-auflösender Mediator zur Verwendung nach Anspruch 9, wobei die Behandlungsstelle die äußere Vulva umfasst.

11. Pro-auflösender Mediator zur Verwendung nach einem der Ansprüche 1-10, ferner umfassend ein Verabreichen eines zweiten therapeutischen Mittels an das Subjekt.

12. Pro-auflösender Mediator zur Verwendung nach Anspruch 11, wobei das zweite therapeutische Mittel ein zweiter Pro-auflösender Mediator ist.

13. Pro-auflösender Mediator zur Verwendung nach Anspruch 11, wobei das zweite therapeutische Mittel ein antimikrobielles Mittel oder ein antivirales Mittel ist.

## Revendications

1. Médiateur pro-résolution destiné à être utilisé dans un procédé de réduction ou de prévention de l'irritation du tractus génital inférieur chez un sujet féminin, le procédé comprenant l'administration d'une quantité efficace du médiateur pro-résolution par voie topique à un site de traitement du tractus génital inférieur du sujet,
dans lequel l'irritation est associée à une affection inflammatoire du tractus génital choisie dans le groupe constitué par la vulvodynie provoquée localisée (VPL), le lichen plan, le lichen scléro-atrophique, la vaginite inflammatoire desquamative, la vulvo-vaginite atrophique associée au cancer du sein et le prurit chronique, et
dans lequel le médiateur pro-résolution est (1) choisi dans le groupe constitué par Resolvin D₂, Resolvin D₃, Resolvin D₄, Resolvin D₅, Resolvin E₁, Maresin-1, epi-Maresin-1, Lipoxin A₄, Protectin D1, Protectin DX et DHA ; ou (2) un mélange comprenant DHA, 14-HDHA, 17-HDHA et 18-HEPE.

2. Médiateur pro-résolution destiné à être utilisé selon la revendication 1, dans lequel le médiateur pro-résolution est la Maresin-1.

3. Médiateur pro-résolution destiné à être utilisé selon la revendication 1, dans lequel le médiateur pro-résolution est la Protectine D1.

4. Médiateur pro-résolution destiné à être utilisé selon l'une quelconque des revendications 1 à 3, dans lequel l'affection inflammatoire du tractus génital est la VPL.

5. Médiateur pro-résolution destiné à être utilisé selon l'une quelconque des revendications 1 à 4, dans lequel le médiateur pro-résolution est administré à une dose de 0,0001 mg/kg à 100 mg/kg.

6. Médiateur pro-résolution destiné à être utilisé selon l'une quelconque des revendications 1 à 5, dans lequel le médiateur pro-résolution est administré une fois par semaine, 2 à 3 fois par semaine, une fois par jour, deux fois par jour ou trois fois par jour.

7. Médiateur pro-résolution destiné à être utilisé selon l'une quelconque des revendications 1 à 6, dans lequel le médiateur pro-résolution est administré avant que le sujet ne soit exposé à une stimulation secondaire provoquant une irritation.

8. Médiateur pro-résolution destiné à être utilisé selon l'une quelconque des revendications 1 à 7, dans lequel le site de traitement comprend le vestibule vulvaire.

9. Médiateur pro-résolution destiné à être utilisé selon l'une quelconque des revendications 1 à 8, dans lequel le site de traitement comprend la vulve externe, le vestibule ou le vagin.

10. Médiateur pro-résolution destiné à être utilisé selon la revendication 9, dans lequel le site de traitement comprend la vulve externe.

11. Médiateur pro-résolution destiné à être utilisé selon l'une quelconque des revendications 1 à 10, comprenant en outre l'administration d'un second agent thérapeutique au sujet.

12. Médiateur pro-résolution destiné à être utilisé selon la revendication 11, dans lequel le second agent thérapeutique est un second médiateur pro-résolution.

13. Médiateur pro-résolution destiné à être utilisé selon la revendication 11, dans lequel le second agent thérapeutique est un agent antimicrobien ou un agent antiviral.
